(19)

Europäisches
Patentamt
European
Patent Office
Office européen
des brevets

(11) **EP 2 498 302 A1**

(12) **EUROPEAN PATENT APPLICATION**
published in accordance with Art. 153(4) EPC

(43) Date of publication:
**12.09.2012 Bulletin 2012/37**

(21) Application number: **10828242.7**

(22) Date of filing: **28.10.2010**

(51) Int Cl.:
***H01L 31/10*** *(2006.01)*　　　***C07K 17/02*** *(2006.01)*

(86) International application number:
**PCT/JP2010/069197**

(87) International publication number:
**WO 2011/055682 (12.05.2011 Gazette 2011/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.11.2009 JP 2009252778**

(71) Applicant: **Sony Corporation
Tokyo 108-0075 (JP)**

(72) Inventors:
 • **LUO Wei
 Tokyo 108-0075 (JP)**

 • **GOTO Yoshio
 Tokyo 108-0075 (JP)**
 • **YAMADA Seiji
 Tokyo 108-0075 (JP)**
 • **TOKITA Yuichi
 Tokyo 108-0075 (JP)**

(74) Representative: **Müller, Achim Fritz
 Müller Hoffmann & Partner
 Patentanwälte
 Innere Wiener Straße 17
 D-81667 München (DE)**

(54) **MULTILAYER TRANSPARENT LIGHT-RECEIVING ELEMENT AND ELECTRONIC DEVICE**

(57)　 A multilayer transparent light-receiving device with significantly high photoresponsive speed that is easily manufactured, and a high-performance electronic device using the multilayer transparent light-receiving device are provided. The multilayer transparent light-receiving device is composed by laminating a plurality of protein transparent light-receiving elements 1 using an electron transfer protein. The protein transparent light-receiving element 1 has a structure in which a transparent substrate, a transparent electrode, an electron transfer protein layer, an electrolyte layer, and a transparent counter electrode are sequentially laminated. The multilayer transparent light-receiving device is used as a light-receiving device for a camera, an optical disc system and the like.

FIG. 1

EP 2 498 302 A1

**Description**

Technical Field

The present invention relates to a multilayer transparent light-receiving device and an electronic device, and in particular, relates to a multilayer transparent light-receiving device using a protein and various electronic devices such as a three dimensional display, a three dimensional image sensor, and a camera using the multilayer transparent light-receiving device as a light detector or the like.

Background Art

In the past, a CCD, a CMOS and the like have been mainly used as a light-receiving device. However, since the CCD, the CMOS and the like are structured based on silicon semiconductor technology, the light-receiving device itself has not been transparent. Thus, most stereoscopic view cameras using the foregoing existing light-receiving devices use binocular parallax simulating mechanism similar to human eyes (for example, a stereo camera or the like). However, by using such mechanism, two or more cameras should be connected, and the structure becomes complicated. Further, two or more lenses should be prepared naturally, and thus it is difficult to downsize the cameras. Further, since one eye is available for one focus of an image pickup section, it has not be possible to obtain a picture in which various objects are focused on concurrently. Further, in the case where a near place is captured immediately after a state that a significantly far place is focused on, since one eye is available for only one focus, the lens should be moved largely for focusing at high velocities, which limits ability for focusing at extremely high speeds.

Meanwhile, in optical disc systems, optical discs have been progressively multilayered, which contributes to significant improvement of the recording capacity. However, in the foregoing existing light-receiving device, it is not possible to achieve a multilayered light-receiving device for light detection in the optical disc system, which is an obstacle in the way of development of the optical disc system using the multilayered optical disc.

In the past, a light transmissive image recognition device capable of transmitting an inputted image has been proposed (see Patent document 1). The light transmissive image recognition device includes a first transparent substrate in which a plurality of transparent pixel electrodes are formed on the surface two dimensionally, a second transparent substrate in which a transparent opposed electrode is formed on the surface, and a visual substance analogous protein orientation alignment film layer and a transparent insulating layer arranged between both electrodes. As the visual substance analogous protein orientation alignment film layer, a bacteriorhodopsin orientation alignment film layer is used.

In addition, as a photoelectric conversion element using a protein, a photoelectric conversion device using a protein immobilized electrode in which zinc-substituted horse heart cytochrome c (obtained by substituting iron as a central metal of prosthetic group heme of horse heart cytochrome c with zinc) is immobilized onto a gold electrode has been proposed (see Patent document 2). In addition, it is disclosed that a photocurrent is obtained by the protein immobilized electrode.

Citation List

Patent documents

Patent document 1: Japanese Unexamined Patent Application Publication No. 2002-334986
Patent document 2: Japanese Unexamined Patent Application Publication No. 2007-220445

Nonpatent documents

Nonpatent document 1: McLendon G. and Smith M. J. Biol. Chem. 253, 4004 (1978)
Nonpatent document 2: Moza B. and two others, Biochim. Biophys. Acta1646, 49 (2003)
Nonpatent document 3: Vanderkooi, J. M. and two others, Eur. J. Biochem. 64, 381-387 (1976)
Nonpatent document 4: Tokita, Y. and four others, J. Am. Chem. Soc. 130, 5302 (2008)
Nonpatent document 5: Gouterman M., Optical spectra and electronic structure of porphyrins and related rings, in "The Porphyrins," Vol. 3, Dolphin D. ed., pp. 1-156, Academic Press (1978)

Summary of the Invention

However, in the light transmissive image recognition device proposed in Patent document 1, when an image is projected from the first transparent substrate side onto the visual substance analogous protein orientation alignment film layer, an induction current induced to the pixel electrode by electric polarization of the visual substance analogous protein orientation alignment film layer is detected. Thus, the photoresponsive speed is slow, and in addition, since Langmuir Blodgett method is used for forming the visual substance analogous protein orientation alignment film layer, productivity is not favorable. Further, in Patent document 1, detection of the induction current induced to the pixel electrode by electric polarization of the visual substance analogous protein orientation alignment film layer is not verified at all.

Therefore, the problems to be solved by the present invention are to provide a multilayer transparent light-receiving device with significantly high photoresponsive speed and being easily manufactured, and a high-performance electronic device using the multilayer transparent light-receiving device.

To solve the foregoing problems, in the present invention, there is provided a multilayer transparent light-receiving device that has a plurality of protein transparent light-receiving elements laminated on each other using an electron transfer protein.

Further, in the present invention, there is provided an electronic device that includes a multilayer transparent light-receiving device that has a plurality of protein transparent light-receiving elements laminated on each other using an electron transfer protein.

In the present invention, as the electron transfer protein, existing known electron transfer proteins are able to be used. More specifically, as the electron transfer protein, an electron transfer protein containing a metal or an electron transfer protein not containing a metal (metal free electron transfer protein) is able to be used. The metal contained in the electron transfer protein is suitably a transition metal having electrons in a high energy orbit equal to or greater than d orbit (for example, zinc, iron or the like). As the electron transfer protein, a new electron transfer protein described later is able to be used.

The electron transfer protein is typically immobilized onto a transparent electrode made of a material transparent to light to be received, typically visible light. In a typical example, the protein transparent light-receiving element has a protein immobilized electrode in which the electron transfer protein is immobilized onto the transparent electrode and a counter electrode. In another typical example, the protein transparent light-receiving element has a structure in which a solid protein layer composed of the electron transfer protein is sandwiched between a first transparent electrode and a second transparent electrode. As a material of the transparent electrode, both an inorganic material and an organic material may be used, and the material is selected according to needs.

As the electronic device, any type may be applicable as long as the multilayer transparent light-receiving device is able to be used. Specific examples thereof include a three dimensional display, a three dimensional image sensor, a camera, and an optical recording reproduction system.

In the present invention configured as above, the electron transfer protein has higher photoresponsive speed than a visual substance analogous protein such as bacteriorhodopsin. In addition, for example, by coating the transparent electrode with a solution containing the electron transfer protein, the protein immobilized electrode is able to be more easily fabricated.

According to the present invention, a multilayer transparent light-receiving device that has significantly high photoresponsive speed and that is easily manufactured is able to be achieved. Further, by using such a superior multilayer transparent light-receiving device, a high performance electronic device is able to be achieved.

Brief Description of Drawings

[FIG. 1] FIG. 1 is a schematic diagram illustrating a multilayer transparent light-receiving device according to a first embodiment of the present invention.

[FIG. 2] FIG. 2 is a cross sectional view illustrating a protein transparent light-receiving element composing the multilayer transparent light-receiving device according to the first embodiment of the present invention.

[FIG. 3] FIG. 3 is a schematic diagram illustrating a first example of usage modes of the protein transparent light-receiving element composing the multilayer transparent light-receiving device according to the first embodiment of the present invention.

[FIG. 4] FIG. 4 is a schematic diagram illustrating a second example of usage modes of the protein transparent light-receiving element composing the multilayer transparent light-receiving device according to the first embodiment of the present invention.

[FIG. 5] FIG. 5 is a schematic diagram illustrating a third example of usage modes of the protein transparent light-receiving element composing the multilayer transparent light-receiving device according to the first embodiment of the present invention.

[FIG. 6] FIG. 6 is a schematic diagram illustrating measurement result of ultraviolet visible absorption spectrum of tin-substituted horse heart cytochrome c used in a protein transparent light-receiving element composing a multilayer transparent light-receiving device according to a second embodiment of the present invention.

[FIG. 7] FIG. 7 is a schematic diagram illustrating measurement result of ultraviolet visible absorption spectrum of tin-substituted bovine heart cytochrome c used in the protein transparent light-receiving element composing the multilayer transparent light-receiving device according to the second embodiment of the present invention.

[FIG. 8] FIG. 8 is a schematic diagram illustrating measurement result of ultraviolet visible absorption spectrum of zinc-substituted horse heart cytochrome c.

[FIG. 9] FIG. 9 is a schematic diagram illustrating measurement result of ultraviolet visible absorption spectrum of zinc-substituted bovine heart cytochrome c.

[FIG. 10] FIG. 10 is a schematic diagram illustrating measurement result of temporal change of the ultraviolet visible absorption spectrum of the tin-substituted horse heart cytochrome c used in the protein transparent light-receiving element composing the multilayer transparent light-receiving device according to the second embodiment of the present invention.

[FIG. 11] FIG. 11 is a schematic diagram illustrating measurement result of temporal change of the ultraviolet visible absorption spectrum of the tin-substituted bovine heart cytochrome c used in the protein transparent light-receiving element composing the multilayer transparent light-receiving device according to the second embodiment of the present invention.

[FIG. 12] FIG. 12 is a schematic diagram illustrating measurement result of temporal change of the ultraviolet visible absorption spectrum of the zinc-substituted horse heart cytochrome c.

[FIG. 13] FIG. 13 is a schematic diagram illustrating measurement result of temporal change of the ultraviolet visible absorption spectrum of the zinc-substituted bovine heart cytochrome c.

[FIG. 14] FIG. 14 is a schematic diagram illustrating an example of fitting of second-order reaction formula of photodegradation reaction of the tin-substituted horse heart cytochrome c and the tin-substituted bovine heart cytochrome c used in the protein transparent light-receiving device composing the multilayer transparent light-receiving device according to the second embodiment of the present invention.

[FIG. 15] FIG. 15 is a schematic diagram illustrating an example of fitting of second-order reaction formula of photodegradation reaction of the zinc-substituted horse heart cytochrome c and the zinc-substituted bovine heart cytochrome c.

[FIG. 16] FIG. 16 is a plane view illustrating a protein immobilized electrode used for photocurrent generation experiment of the metal substitution cytochrome c in the second embodiment of the present invention.

[FIG. 17] FIG. 17 is a schematic diagram illustrating measurement result of photocurrent action spectrum of the protein immobilized electrode illustrated in FIG. 16.

[FIG. 18] FIG. 18 is a schematic diagram illustrating average value of Soret band photocurrent values of the protein immobilized electrode illustrated in FIG. 16.

[FIG. 19] FIG. 19 is a schematic diagram illustrating measurement result of ultraviolet visible absorption spectrums of various metal substitution cytochromes c.

[FIG. 20] FIG. 20 is a schematic diagram illustrating measurement result of fluorescent spectrums of various metal substitution cytochromes c.

[FIG. 21] FIG. 21 is a schematic diagram illustrating integral fluorescent intensity to absorbance in wavelength 409 nm of the tin-substituted horse heart cytochrome c and the zinc-substituted horse heart cytochrome c.

[FIG. 22] FIG. 22 is a schematic diagram illustrating integral fluorescent intensity to absorbance in wavelength 409 nm of the tin-substituted bovine heart cytochrome c, the zinc-substituted bovine heart cytochrome c, and the zinc-substituted horse heart cytochrome c.

[FIG. 23] FIG. 23 is a cross sectional view illustrating a non-wetted all solid protein transparent light-receiving element composing a multilayer transparent light-receiving device according to a fourth embodiment of the present invention.

[FIG. 24] FIG. 24 is a cross sectional view illustrating an enlarged main section of the non-wetted all solid protein transparent light-receiving element illustrated in FIG. 23.

[FIG. 25] FIG. 25 is a schematic diagram for explaining operation of the non-wetted all solid protein transparent light-receiving element composing the multilayer transparent light-receiving device according to the fourth embodiment of the present invention.

[FIG. 26] FIG. 26 is a plane view for explaining a manufacturing method of a non-wetted all solid protein transparent light-receiving element composing a multilayer transparent light-receiving device according to an example of the present invention.

[FIG. 27] FIG. 27 is a plane view for explaining the manufacturing method of the non-wetted all solid protein transparent light-receiving element composing the multilayer transparent light-receiving device according to the example of the present invention.

[FIG. 28] FIG. 28 is a cross sectional view illustrating the non-wetted all solid protein transparent light-receiving element composing the multilayer transparent light-receiving device according to the example of the present invention.

[FIG. 29] FIG. 29 is a schematic diagram illustrating measurement result of photocurrent action spectrum of the non-wetted all solid protein transparent light-receiving element composing the multilayer transparent light-receiving device according to the example of the present invention.

[FIG. 30] FIG. 30 is a schematic diagram illustrating measurement result of background current-voltage characteristics of the non-wetted all solid protein transparent light-receiving element composing the multilayer transparent light-receiving device according to the example of the present invention.

[FIG. 31] FIG. 31 is a schematic diagram illustrating measurement result of current-voltage characteristics of the non-wetted all solid protein transparent light-receiving element composing the multilayer transparent light-receiving device according to the example of the present invention.

[FIG. 32] FIG. 32 is a schematic diagram illustrating measurement result of photocurrent action spectrum of the non-wetted all solid protein transparent light-receiving element composing the multilayer transparent light-receiving device according to the example of the present invention and a liquid type protein transparent light-receiving element.

[FIG. 33] FIG. 33 is a schematic diagram obtained by normalizing the measurement result of the photocurrent action spectrums of the non-wetted all solid protein transparent light-receiving element composing the multilayer transparent light-receiving device according to the example of the present invention and the liquid type protein transparent light-receiving element so that the photocurrent peak value becomes 1.

[FIG. 34] FIG. 34 is a schematic diagram illustrating measurement result of light degradation curves of the non-wetted all solid protein transparent light-receiving element composing the multilayer transparent light-receiving device according to the example of the present invention and the liquid type protein transparent light-receiving element.

[FIG. 35] FIG. 35 is a schematic diagram obtained by normalizing the measurement result of light degradation curves of the non-wetted all solid protein transparent light-receiving element composing the multilayer transparent light-receiving device according to the example of the present invention and the liquid type protein transparent light-receiving element so that the photocurrent peak value at the start of irradiation becomes 1.

[FIG. 36] FIG. 36 is a schematic diagram illustrating measurement result of frequency response of the liquid type protein transparent light-receiving element.

[FIG. 37] FIG. 37 is a schematic diagram illustrating measurement result of frequency response of the non-wetted all solid protein transparent light-receiving element composing the multilayer transparent light-receiving device according to the example of the present invention.

[FIG. 38] FIG. 38 is a schematic diagram illustrating measurement result of photocurrent action spectrum of the non-wetted all solid protein transparent light-receiving element composing the multilayer transparent light-receiving device according to the example of the present invention.

[FIG. 39] FIG. 39 is a schematic diagram illustrating measurement result of light degradation curves of the non-wetted all solid protein transparent light-receiving element composing the multilayer transparent light-receiving device according to the example of the present invention.

[FIG. 40] FIG. 40 is a schematic diagram illustrating a multilayer transparent light-receiving device according to a fifth embodiment of the present invention.

[FIG. 41] FIG. 41 is a schematic diagram illustrating a multilayer transparent light-receiving device according to a sixth embodiment of the present invention.

[FIG. 42] FIG. 42 is a schematic diagram for explaining a stereoscopic imaging system according to a seventh embodiment of the present invention.

[FIG. 43] FIG. 43 is a schematic diagram for explaining the stereoscopic imaging system according to the seventh embodiment of the present invention.

[FIG. 44] FIG. 44 is a schematic diagram for explaining the stereoscopic imaging system according to the seventh embodiment of the present invention.

[FIG. 45] FIG. 45 is a schematic diagram for explaining the stereoscopic imaging system according to the seventh embodiment of the present invention.

[FIG. 46] FIG. 46 is a schematic diagram for explaining the stereoscopic imaging system according to the seventh embodiment of the present invention.

[FIG. 47] FIG. 47 is a schematic diagram for explaining the stereoscopic imaging system according to the seventh embodiment of the present invention.

[FIG. 48] FIG. 48 is a schematic diagram for explaining the stereoscopic imaging system according to the seventh embodiment of the present invention.

[FIG. 49] FIG. 49 is a schematic diagram for explaining the stereoscopic imaging system according to the seventh embodiment of the present invention.

[FIG. 50] FIG. 50 is a schematic diagram for explaining the stereoscopic imaging system according to the seventh embodiment of the present invention.

[FIG. 51] FIG. 51 is a schematic diagram for explaining the stereoscopic imaging system according to the seventh embodiment of the present invention.

[FIG. 52] FIG. 52 is a schematic diagram for explaining a stereoscopic imaging system according to an eighth embodiment of the present invention.

[FIG. 53] FIG. 53 is a schematic diagram for explaining a stereoscopic imaging system according to a ninth embodiment of the present invention.

[FIG. 54] FIG. 54 is a schematic diagram illustrating an optical disc system according to a tenth embodiment of the present invention.

[FIG. 55] FIG. 55 is a schematic diagram illustrating an optical recording reproduction system according to an eleventh embodiment of the present invention.

Description of Embodiments

Embodiments for carrying out the present invention (hereinafter referred to as "embodiment") will be hereinafter described. In addition, the description will be given in the following order.

1. First embodiment (multilayer transparent light-receiving device)
2. Second embodiment (multilayer transparent light-receiving device)
3. Third embodiment (multilayer transparent light-receiving device)
4. Fourth embodiment (multilayer transparent light-receiving device)
5. Fifth embodiment (multilayer transparent light-receiving device)
6. Sixth embodiment (multilayer transparent light-receiving device)
7. Seventh embodiment (stereoscopic imaging system)
8. Eighth embodiment (stereoscopic imaging system)
9. Ninth embodiment (stereoscopic imaging system)
10. Tenth embodiment (optical disc system)
11. Eleventh embodiment (optical recording reproduction system)

<1. First embodiment>

[Multilayer transparent light-receiving device]

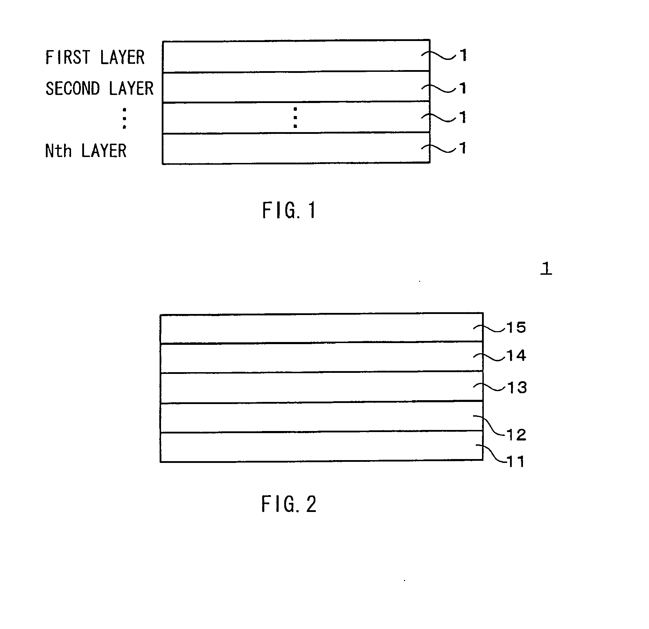

FIG. 1 illustrates a multilayer transparent light-receiving device according to a first embodiment.

As illustrated in FIG. 1, the multilayer transparent light-receiving device is composed of N layers (N is an integer number equal to or greater than 2) of a protein transparent light-receiving element 1 laminated on each other. The number of layers N of the protein transparent light-receiving element 1 is able to be selected as appropriate according to the purpose of the multilayer transparent light-receiving device. Further, the planar shape, the size, and the thickness of the multilayer transparent light-receiving device and the protein transparent light-receiving element 1 are able to be selected as appropriate. Though the thickness of the protein transparent light-receiving element 1 is generally, for example, from 10 $\mu$m to 1 mm both inclusive, the thickness of the protein transparent light-receiving element 1 is not limited thereto.

FIG. 2 illustrates a structural example of the protein transparent light-receiving element 1.

As illustrated in FIG. 2, in the protein transparent light-receiving element 1, an electron transfer protein layer 13 is immobilized onto a transparent electrode 12 provided on a transparent substrate 11. A transparent counter electrode 15 is provided to be placed opposite the electron transfer protein layer 13 with an electrolyte layer 14 in between. The electron transfer protein layer 13 is composed of a monomolecular film or a multimolecular film of an electron transfer protein. Each electron transfer protein of the electron transfer protein layer 13 may be directly immobilized onto the transparent electrode 12, or indirectly immobilized onto the transparent electrode 12 with an intermediate layer such as a self-assembled monomolecular film in between. The electrolyte layer 14 is composed of an electrolyte solution or a solid electrolyte. In order to prevent the electrolyte layer 14 from being leaked outside, being contacted with air, or being dried, the surrounding of the electrolyte layer 14 is suitably sealed by a sealing wall (not illustrated). Otherwise, the entire protein transparent light-receiving element 1 may be contained in a transparent container in some cases.

In FIG. 2, each layer composing the protein transparent light-receiving element 1 is illustrated to have a flat surface shape. However, each surface shape of each layer is optional, and any shape such as a concave face, a convex face, and a convexoconcave face may be adopted. In particular, the electron transfer protein layer 13 is able to be easily immobilized onto the transparent electrode 12 without relation to the surface shape of the transparent electrode 12.

As a material of the transparent substrate 11, for example, various inorganic or organic transparent materials such as glass, mica, and polyethylene terephthalate (PET) are able to be used.

As a material of the transparent electrode 12, a transparent metal oxide such as ITO (indium-tin composite oxide), FTO (fluorine-doped tin oxide), and NESA glass ($SnO_2$ glass), an extremely thin metal film capable of transmitting light such as a Au film and the like are able to be used.

As an electron transfer protein of the electron transfer protein layer 13, specifically, for example, cytochromes, iron-sulfur proteins, blue-copper proteins and the like are able to be used. Examples of the cytochromes include cytochrome c (zinc-substituted cytochrome c, metal free cytochrome c and the like), cytochrome b, cytochrome b5, cytochrome cl, cytochrome a, cytochrome a3, cytochrome f, and cytochrome b6. Examples of the iron-sulfur proteins include rubredoxin, two-iron ferredoxin, three-iron ferredoxin, and four-iron ferredoxin. Examples of the blue-copper proteins include plastocyanin, azurin, pseudo azurin, plantacyanin, steracyanin, and amicyanin. The electron transfer protein is not limited thereto. For example, a derivative of the foregoing electron transfer proteins (obtained by chemically modifying an amino-acid residue of a skeleton) or a variant thereof (obtained by substituting part of an amino-acid residue of a skeleton with other amino-acid residue) is able to be used. These electron transfer proteins are all water-soluble proteins.

The protein transparent light-receiving element 1 is able to be operated both in a solution (electrolyte solution) and in dry environment as long as photoelectric conversion function and electron transfer function of the electron transfer protein of the electron transfer protein layer 13 are not impaired. In other words, the electrolyte layer 14 may be composed of an electrolyte solution or a solid electrolyte. As an electrolyte of the electrolyte layer 14 (or redox species), an electrolyte with which oxidation reaction is initiated in the protein immobilized electrode in which the electron transfer protein layer 13 is immobilized onto the transparent electrode 12 and reduction reaction is initiated in the transparent counter electrode 15, or an electrolyte with which reduction reaction is initiated in the foregoing protein immobilized electrode and oxidation reaction is initiated in the transparent counter electrode 15 is used. Specifically, as the electrolyte, for example, $K_4$[Fe$(CN)_6$], [Co$(NH_3)_6$]Cl$_3$ or the like is used. In the case where the protein transparent light-receiving element 1 is operated in dry environment, typically, for example, the electrolyte layer 14 composed of a solid electrolyte not absorbing the electron transfer protein, specifically the electrolyte layer 14 composed of a wet solid electrolyte such as agar and polyacrylamide gel is sandwiched between the protein immobilized electrode and the transparent counter electrode 15, and the surrounding thereof is provided with a sealing wall to prevent the solid electrolyte from being dried. In these cases, a photocurrent is able to be obtained in the case where light is received at a light receiving section composed of the electron transfer protein layer 13 under polarity based on natural electrode potential difference between the protein immobilized electrode and the transparent counter electrode 15.

As a material of the transparent counter electrode 15, a transparent metal oxide such as ITO, FTO, and NESA glass, an extremely thin metal film capable of transmitting light such as a Au film and the like are able to be used.

[Usage mode of the protein transparent light-receiving element 1]

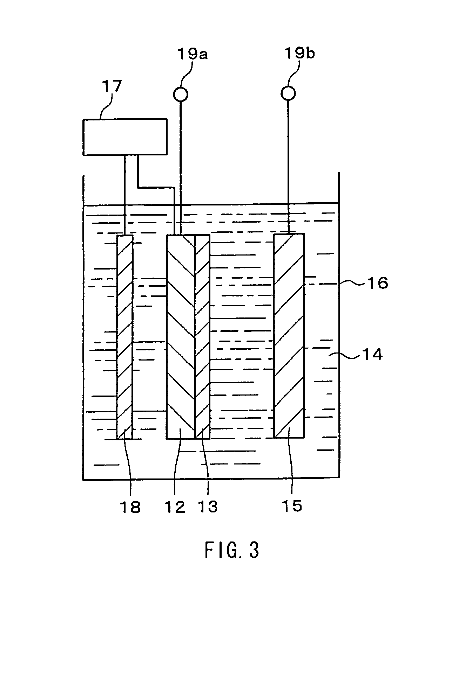

FIG. 3 illustrates a first example of usage modes of the protein transparent light-receiving element 1.
As illustrated in FIG. 3, in the first example, the protein immobilized electrode in which the electron transfer protein layer 13 is immobilized onto the transparent electrode 12 and the transparent counter electrode 15 are placed opposite each other. The protein immobilized electrode and the transparent counter electrode 15 are soaked in the electrolyte layer 14 composed of an electrolyte solution contained in a transparent container 16. As the electrolyte solution, a solution not impairing electron transfer protein function of the electron transfer protein layer 13 is used. Further, as an electrolyte of the electrolyte solution (or redox species), an electrolyte with which oxidation reaction is initiated in the protein immobilized electrode and reduction reaction is initiated in the transparent counter electrode 15, or an electrolyte with which reduction reaction is initiated in the protein immobilized electrode and oxidation reaction is initiated in the transparent counter electrode 15 is used.

For performing photoelectric conversion by the protein transparent light-receiving element 1, the electron transfer protein layer 13 of the protein immobilized electrode is irradiated with light in a state that a bias voltage is applied to the protein immobilized electrode with reference to a transparent reference electrode 18 by a bias power source 17. The light is monochromatic light capable of light excitation of the electron transfer protein of the electron transfer protein layer 13 or light having an element of such light. In this case, by adjusting at least one of the bias voltage applied to the protein immobilized electrode, the intensity of the irradiated light, and the wavelength of the irradiated light, the magnitude and/or the polarity of a photocurrent flown through the device is able to be changed. The photocurrent is extracted outside from terminals 19a and 19b.

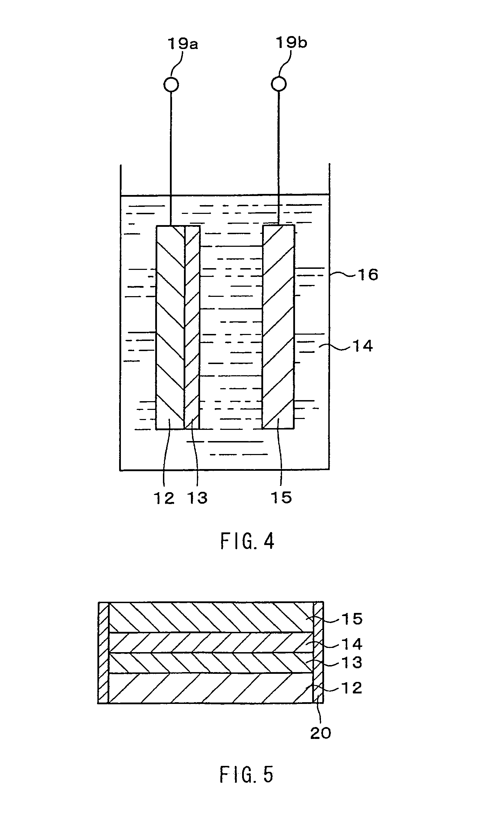

FIG. 4 illustrates a second example of usage modes of the protein transparent light-receiving element 1.
As illustrated in FIG. 4, in the second example, differently from the first example, a bias voltage is not generated by using the bias power source 17, and natural electrode potential difference between the protein immobilized electrode and the transparent counter electrode 15 is used as a bias voltage. In this case, it is not necessary to use the transparent reference electrode 18. Thus, the protein transparent light-receiving element 1 is a two electrode system using the protein immobilized electrode and the transparent counter electrode 15. Except for the foregoing characteristics, the second example has a structure similar to that of the first example.

FIG. 5 illustrates a third example of usage modes of the protein transparent light-receiving element 1. The protein transparent light-receiving element 1 according to the third example is able to be operated in dry environment, while the protein transparent light-receiving devices 1 according to the first and the second examples are operated in a solution.

As illustrated in FIG. 5, in the protein transparent light-receiving element 1, the electrolyte layer 14 made of a solid electrolyte is sandwiched between the protein immobilized electrode and the transparent counter electrode 15. Further, a sealing wall 20 for preventing the solid electrolyte from being dried is provided to encircle the surrounding of the electrolyte layer 14. As the solid electrolyte, a solid electrolyte not impairing electron transfer protein function of the electron transfer protein layer 13 is used. Specifically, agar, polyacrylamide gel or the like not absorbing the electron transfer protein is used. For performing photoelectric conversion by the protein transparent light-receiving element 1, the natural electrode potential difference between the protein immobilized electrode and the transparent counter electrode 15 is used as a bias voltage, and the electron transfer protein layer 13 of the protein immobilized electrode is irradiated with light. The light is monochromatic light capable of light excitation of the electron transfer protein of the electron transfer protein layer 13 or light having an element of such light. In this case, by adjusting at least one of the natural electrode potential difference between the protein immobilized electrode and the transparent counter electrode 15, the intensity of the irradiated light, and the wavelength of the irradiated light, the magnitude and/or the polarity of a photocurrent flown through the device is able to be changed. Except for the foregoing characteristics, the third example has a structure similar to that of the first example.

[Manufacturing method of multilayer transparent light-receiving device]

A description will be given of an example of a manufacturing method of the multilayer transparent light-receiving device.

First, the transparent electrode 12 formed on the transparent substrate 11 is soaked in a solution containing an electron transfer protein and a buffer solution, and thereby the electron transfer protein is immobilized onto the transparent electrode 12. Thus, the protein immobilized electrode in which the electron transfer protein layer 13 is formed on the transparent electrode 12 is formed.

Next, by using the protein immobilized electrode and the transparent counter electrode 15, the protein transparent light-receiving element 1 illustrated in, for example, FIG. 3, FIG. 4, or FIG. 5 is manufactured.

After that, the necessary number of the protein transparent light-receiving devices are laminated. At this time, the respective protein transparent light-receiving devices 1 are bonded by a transparent adhesive or the like according to needs.

[Operation of multilayer transparent light-receiving device]

When monochromatic light in wavelength corresponding to the electron transfer protein of the electron transfer protein layer 13 or light including the wavelength component enters the electron transfer protein layer 13 of the respective protein transparent light-receiving devices 1 of the multilayer transparent light-receiving device, electrons are generated from the electron transfer protein of the electron transfer protein layer 13 due to light excitation, and the electrons are moved to the transparent electrode 12 due to electron transfer. In the result, a photocurrent is extracted outside from the transparent electrode 12 and the transparent counter electrode 15.

According to the first embodiment, the multilayer transparent light-receiving device in which the plurality of protein transparent light-receiving devices 1 using the electron transfer protein are laminated is able to be achieved. The multilayer transparent light-receiving device is able to be used for various apparatuses, devices and the like that use photoelectric conversion. Specifically, the multilayer transparent light-receiving device is able to be used for an electronic device having a light receiving section and the like. Such an electronic device may be any type fundamentally, and includes a portable type and a stationary type. For example, as will be described later, a camera capable of concurrently focusing on a plurality of objects located in a position different from each other by using one lens is able to be achieved. It shows that the camera is able to obtain information reproducing a three dimensional picture at once with the use of a single lens, which enables realizing a simpler and downsized stereo camera. Further, by using the multilayer transparent light-receiving device, multifocusing and high-speed focusing with the use of a single lens are enabled. Further, in the case where the multilayer transparent light-receiving device is used as a light-receiving device of an optical disc system using a multilayer optical disc or an optical recording reproduction system using a holographic recording medium, parallel readout of the multilayer optical disc and readout of the holographic recording medium are able to be easily performed.

<2. Second embodiment>

[Multilayer transparent light-receiving device]

A multilayer transparent light-receiving device according to a second embodiment has a structure similar to that of the multilayer transparent light-receiving device according to the first embodiment, except that a new electron transfer protein is used as an electron transfer protein of the electron transfer protein layer 13 of the protein transparent light-receiving element 1.
The new electron transfer protein is tin-substituted cytochrome c obtained by substituting iron as a central metal of heme of mammal-derived cytochrome c with tin, or a protein that is composed of an amino-acid sequence obtained by losing, substituting, or adding one or several amino acids in an amino-acid sequence of the mammal-derived cytochrome c and that contains tin. Here, examples of the mammal-derived cytochrome c include horse heart cytochrome c and bovine heart cytochrome c. These new electron transfer proteins have significantly high stability to light irradiation, and are able to retain photoelectric conversion function for a long time.
A description will be given of details and a preparation method of the tin-substituted cytochrome c.

<Tin-substituted cytochrome c>

Table 1 illustrates amino-acid sequences (one letter symbols) of the horse heart cytochrome c (described as CYC HORSE) and the bovine heart cytochrome c (described as CYC BOVIN). As illustrated in Table 1, the horse heart cytochrome c and the bovine heart cytochrome c have the same structure except for three residues out of all 104 amino acid residues. In the bovine heart cytochrome c, Thr48, Lys61, and Thr90 of the horse heart cytochrome c are respectively substituted with Ser48, Gly61, and Gly90.
[Table 1]

```
sp:CYC_HORSE 001 MGDVEKGKKIFVQKCAQCHTVEKGGKHKTG
sp:CYC_BOVIN_001 MGDVEKGKKIFVQKCAQCHTVEKGGKHKTG
                               48
sp:CYC_HORSE_031 PNLHGLFGRKTGQAPGFTYTDANKNKGITW
sp:CYC_BOVIN_031 PNLHGLFGRKTGQAPGFSYTDANKNKGITW
                 61                           90
sp:CYC_HORSE_061 KEETLMEYLENPKKYIPGTKMIFAGIKKKT
sp:CYC_BOVIN_061 GEETLMEYLENPKKYIPGTKMIFAGIKKKG

sp:CYC_HORSE_091 EREDLIAYLKKATNE 104
sp:CYC_BOVIN_091 EREDLIAYLKKATNE 104
```

It has been known that the bovine heart cytochrome c has higher stability of the protein portion to heat and a denaturant (guanidine hydrochloride) than the horse heart cytochrome c (Nonpatent documents 1 and 2). Table 2 illustrates denaturation midpoint temperature $T_{1/2}$ and denaturation midpoint concentration $[Gdn-HCl]_{1/2}$ of the horse heart cytochrome c and the bovine heart cytochrome c. The denaturation midpoint temperature $T_{1/2}$ is the temperature at which the ratio occupied by a denatured protein out of all proteins in the system becomes half (1/2). Further, the denaturation midpoint concentration $[Gdn-HCl]_{1/2}$ is the concentration of guanidine hydrochloride (Gdn-HCl) at which the ratio occupied by the denatured protein out of all proteins in the system becomes half (1/2). As numerical values of $T_{1/2}$ and $[Gdn-HCl]_{1/2}$ are higher, it is more stable.
[Table 2]

|  | $T_{1/2}$ (deg C) | $[Gdn-HCl]_{1/2}$ (M) |
|---|---|---|
| Horse heart cytochrome c | 85 | 2.50 |
| Bovine heart cytochrome c | 85 | 2.61 |

<Preparation of the tin-substituted cytochrome c>

The tin-substituted horse heart cytochrome c and the tin-substituted bovine heart cytochrome c were prepared as described below. For comparative experiment, zinc-substituted horse heart cytochrome c and zinc-substituted bovine heart cytochrome c were also prepared.

The horse heart cytochrome c and the bovine heart cytochrome c made by Sigma Corporation were used.

A description will be mainly given of a preparation method of the tin-substituted horse heart cytochrome c. However, preparation methods of the tin-substituted bovine heart cytochrome c, the zinc-substituted horse heart cytochrome c, and the zinc-substituted bovine heart cytochrome c are similar to the preparation method of the tin-substituted horse heart cytochrome c. In addition, the protein that is composed of the amino-acid sequence obtained by losing, substituting, or adding one or several amino acids in the amino-acid sequence of the horse heart cytochrome c or the bovine heart cytochrome c and that contains tin is also able to be similarly prepared by using a technique such as random mutation and chemical modification as appropriate.

6 mL of 70% fluorinated acid/pyridine was added to 100 mg of horse heart cytochrome c powder, and the resultant was incubated for 10 minutes at room temperature. Thereby, iron as a central metal of heme was removed from the horse heart cytochrome c. 9 mL of 50 mM ammonium acetate buffer solution (pH5.0) was added to the horse heart cytochrome c from which iron was removed as described above. After reaction stop, a metal free horse heart cytochrome c from which the central metal was removed was obtained by gel filtration column chromatography (column volume: 150 mL, resin: Sephadex G-50, developing solvent: 50 mM sodium acetate buffer solution (pH5.0)).

The metal free horse heart cytochrome c solution was condensed as much as possible, and the resultant was added with glacial acetic acid to obtain ph2.5 ($\pm$-0.05). The obtained solution was added with about 25 mg of tin chloride powder, and the resultant was incubated for 30 minutes at 50 deg C under light shielding. In the foregoing process, if zinc acetate or zinc chloride was added instead of tin chloride, a zinc-substituted product was obtained. Ultraviolet visible absorption spectrum was measured every 10 minutes. Until a ratio between absorption peak in wavelength 280 nm of protein and absorption peak in wavelength 408 nm derived from tin porphyrin became constant, incubation was continued.

All operations on and after the foregoing process were performed under light shielding. After the foregoing finally obtained solution was added with saturated diphosphoric acid-sodium hydrogen solution to obtain neutral pH (6.0<), buffer solution exchange to 10 mM sodium phosphate buffer solution (pH 7.0) was performed. After that, a monomeric fraction was collected by cation exchange column chromatography (column volume: 40 mL, resin: SP-Sephadex Fast Flow, elution: linear concentration gradient of 10 to 150 mM sodium phosphate buffer solution (pH 7.0)). Accordingly, the tin-substituted horse heart cytochrome c was prepared.

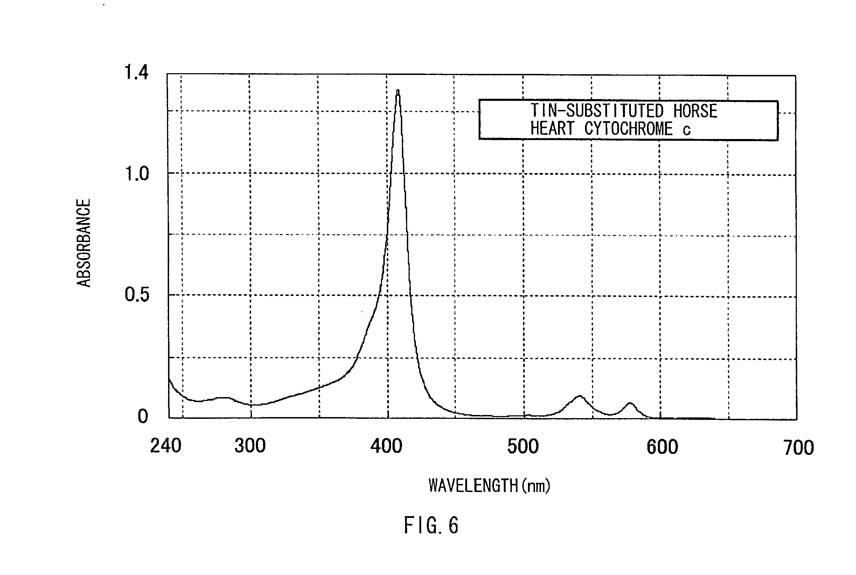

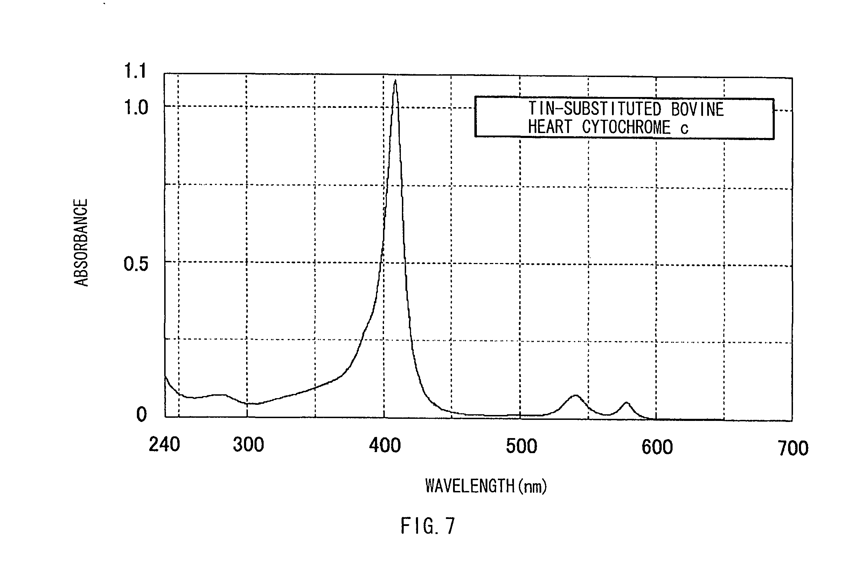

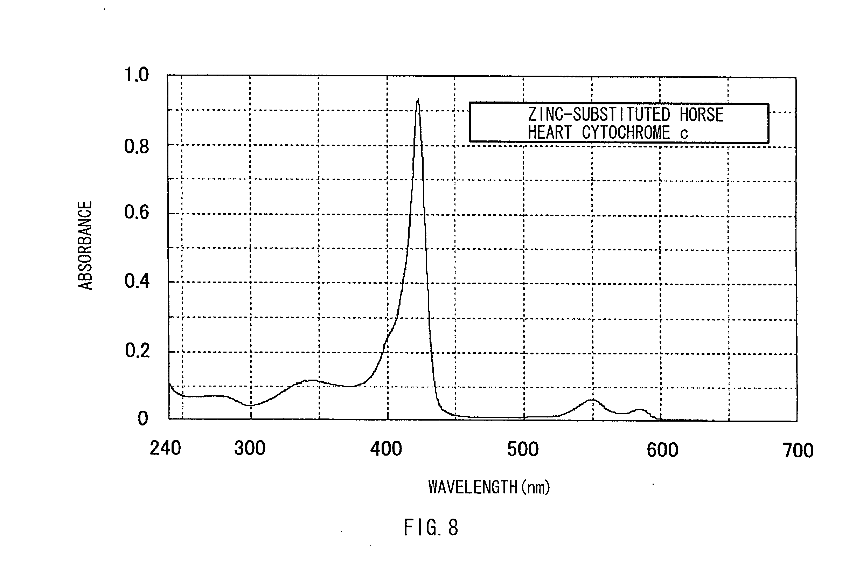

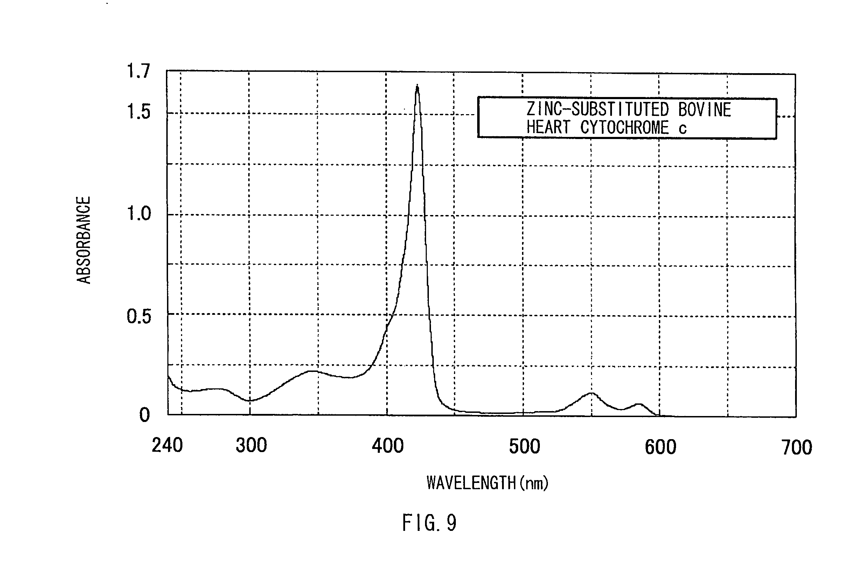

Measurement results of the tin-substituted horse heart cytochrome c, the tin-substituted bovine heart cytochrome c, the zinc-substituted horse heart cytochrome c, and the zinc-substituted bovine heart cytochrome c prepared as described above are illustrated in FIG. 6 to FIG. 9. In the following description, according to needs, the tin-substituted horse heart cytochrome c will be abbreviated to Snhhc, the tin-substituted bovine heart cytochrome c will be abbreviated to Snbvc, the zinc-substituted horse heart cytochrome c will be abbreviated to Znhhc, and the zinc-substituted bovine heart cytochrome c will be abbreviated to Znbvc. As illustrated in FIG. 6 to FIG. 9, the zinc-substituted horse heart cytochrome c and the zinc-substituted bovine heart cytochrome c have the absorption maximum in wavelengths of 280, 346, 423, 550, and 584 nm. Meanwhile, the tin-substituted horse heart cytochrome c and the tin-substituted bovine heart cytochrome c have the absorption maximum in wavelengths of 280, 409, 540, and 578 nm, and do not have $\delta$ band (in the vicinity of 346 nm).

<Light irradiation degradation experiment of the metal substitution cytochromes c>

Light irradiation degradation experiment of the foregoing four types of metal substitution cytochromes c, that is, the tin-substituted horse heart cytochrome c, the tin-substituted bovine heart cytochrome c, the zinc-substituted horse heart cytochrome c, and the zinc-substituted bovine heart cytochrome c was performed as follows.

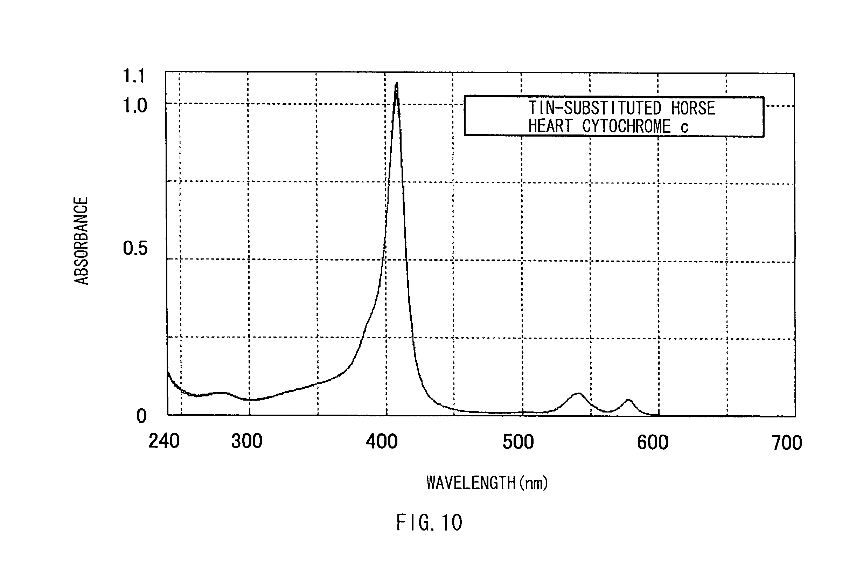

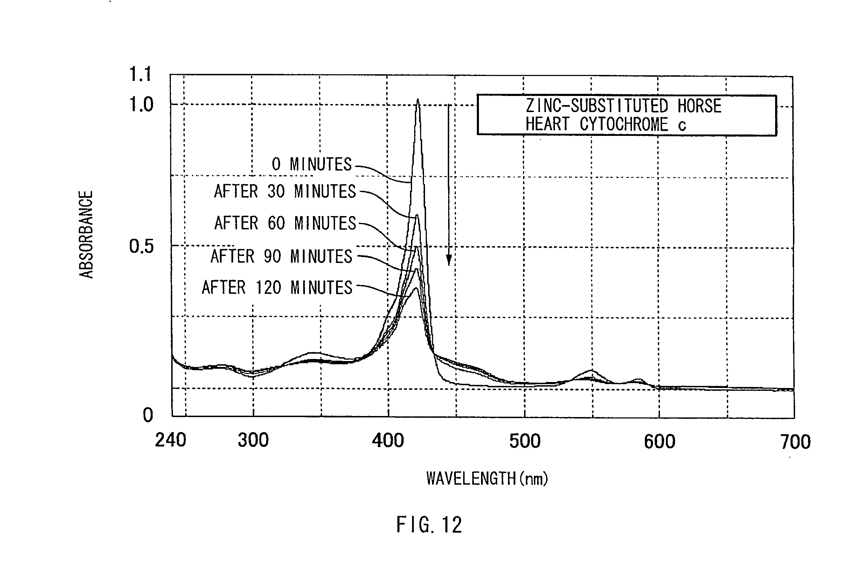

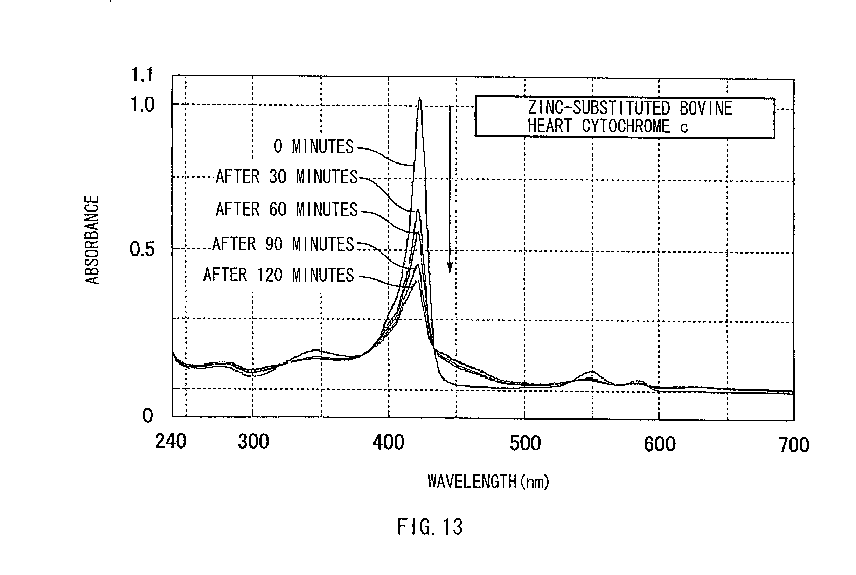

1 mL of about 4 $\mu$M metal substitution cytochrome c (dissolved in 10 mM sodium phosphate buffer solution (pH 7.0)) was set in a cuvette. The zinc-substituted product was irradiated with light in wavelength 420 nm (intensity: 1255 $\mu$W), and the tin-substituted product was irradiated with light in wavelength 408 nm (intensity: 1132 $\mu$W) in a dark room at room temperature. Ultraviolet visible absorption spectrum in wavelengths from 240 to 700 nm both inclusive was measured every 30 minutes. The results thereof are illustrated in FIG. 10 to FIG. 13. Arrows in FIG. 12 and FIG. 13 indicate spectrum change directions.

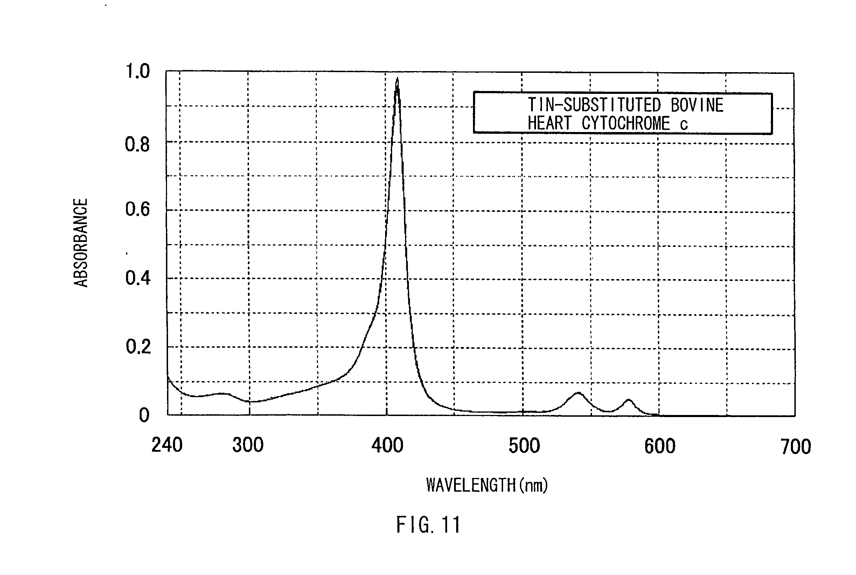

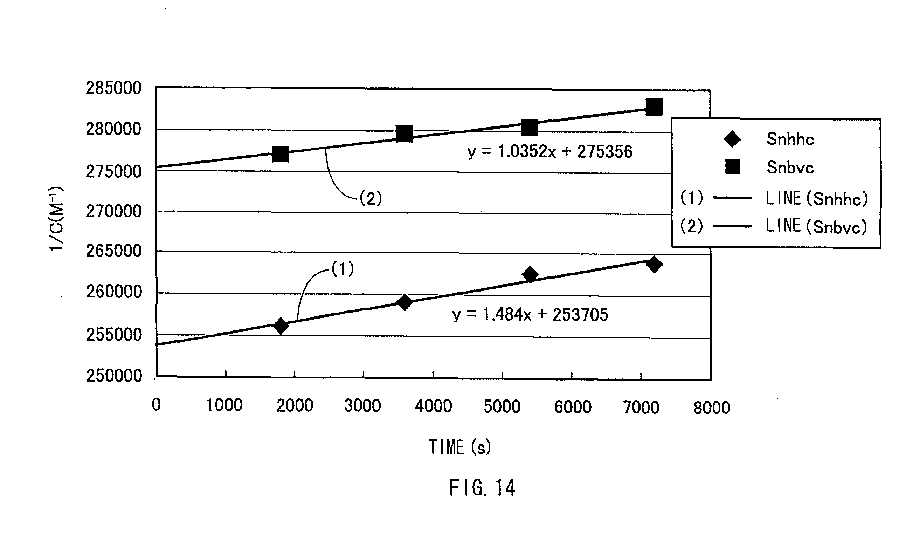

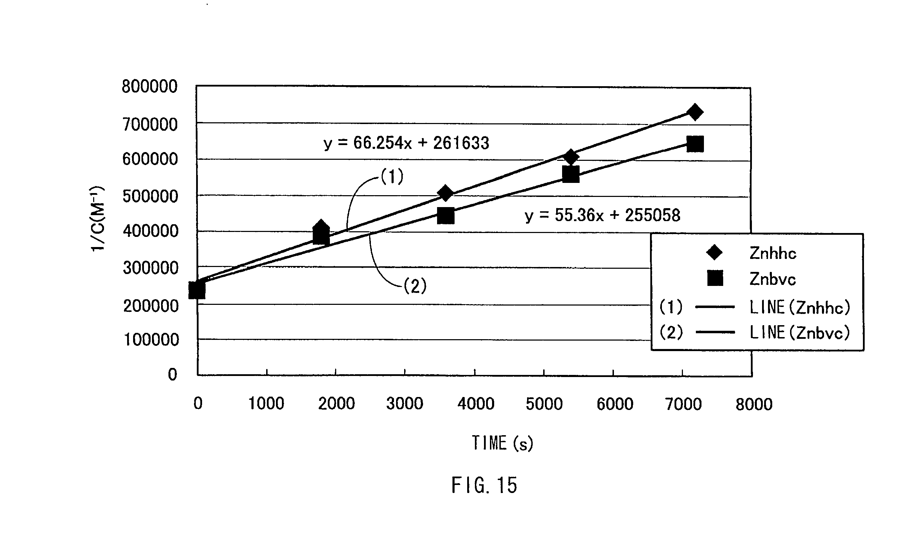

From FIG. 12 and FIG. 13, it was found that in the zinc-substituted horse heart cytochrome c and the zinc-substituted bovine heart cytochrome c, light degradation rapidly proceeded as time advanced. Meanwhile, from FIG. 10 and FIG. 11, it was found that in the tin-substituted horse heart cytochrome c and the tin-substituted bovine heart cytochrome c, spectrums after time advanced almost overlap with initial spectrums, and light degradation was hardly generated after time advanced. Based on absorbance of Soret band (zinc (Zn): 423 nm and tin (Sn): 409 nm) in the ultraviolet visible absorption spectrums illustrated in FIG. 10 to FIG. 13, by using millimole absorbance constant ε (Zn: 243000 $M^{-1}$ $cm^{-1}$, Sn: 267000 $M^{-1}$ $cm^{-1}$, numerical values are quoted from Nonpatent document 3), a concentration (M) was calculated. An inverse thereof was plotted with respect to time (sec (s)). Based on the slope thereof, photodegrative rate constant k was calculated. Inverses (1/C) of concentrations of the tin-substituted horse heart cytochrome c and the tin-substituted bovine heart cytochrome c - time (t) plot is illustrated in FIG. 14, and inverses (1/C) of concentrations of the zinc-substituted horse heart cytochrome c and the zinc-substituted bovine heart cytochrome c - time (t) plot is illustrated in FIG. 15. In FIG. 14 and FIG. 15, a straight line is a fitting curve of second-order reaction formula ($1/C=kt+1/C_0$), where $C_0$ represents an initial concentration, and a slope of the straight line represents the photodegrative rate constant k. In the linear expression indicating the straight lines illustrated in FIG. 14 and FIG. 15, t is indicated by x, and 1/C is indicated by y.

From the average of two experiments, the photodegrative rate constant k of the foregoing four types of metal substitution cytochromes c was obtained. In the result, the photodegrative rate constant k of the tin-substituted horse heart cytochrome c was 1.39 ±0.13 $M^{-1}s^{-1}$, the photodegrative rate constant k of the tin-substituted bovine heart cytochrome c was 0.90±0.20 $M^{-1}s^{-1}$, the photodegrative rate constant k of the zinc-substituted horse heart cytochrome c was 67.2 ±1.4 $M^{-1}s^{-1}$, and the photodegrative rate constant k of the zinc-substituted bovine heart cytochrome c was 56.1±1.0 $M^{-1}s^{-1}$. From the results, it was found that light degradation rates of both the tin-substituted horse heart cytochrome c and the tin-substituted bovine heart cytochrome c were 50 to 60 times slower than those of the zinc-substituted horse heart cytochrome c and the zinc-substituted bovine heart cytochrome c, which meant that both the tin-substituted horse heart cytochrome c and the tin-substituted bovine heart cytochrome c were significantly stable to light irradiation. Further, it was found that both for the zinc-substituted product and the tin-substituted product, light degradation rate of the bovine heart cytochrome c was 1.2 to 1.5 times slower than that of the horse heart cytochrome c, which meant that the bovine heart cytochrome c was stable to light irradiation. In particular, the tin-substituted bovine heart cytochrome c was stable to light irradiation 75 times as much as the zinc-substituted horse heart cytochrome c used in Patent document 1.

<Photocurrent generation experiment of the metal substitution cytochromes c>

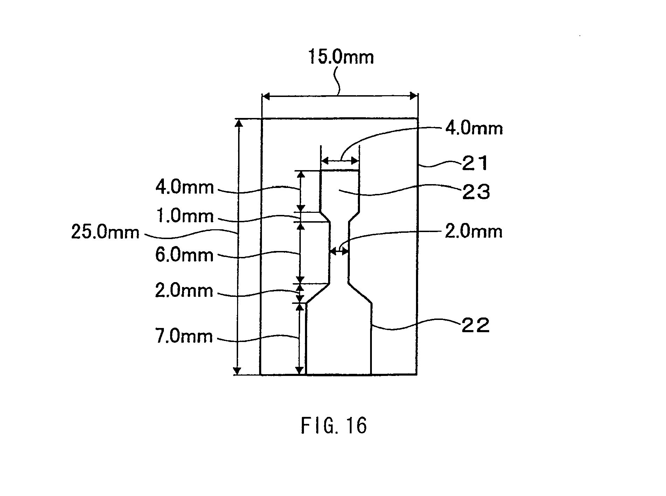

A protein immobilized electrode used for photocurrent generation experiment was fabricated as follows. As illustrated in FIG. 16, an ITO electrode 22 in a given shape was formed on a glass substrate 21 sized 15.0 mm × 25.0 mm being 1 mm thick. Dimensions of respective sections of the ITO electrode 22 were as illustrated in FIG. 16. The thickness of the ITO electrode 22 was 10 nm. The ITO electrode 22 was a working electrode. The size of an irradiation region 23 was 4.0 mm × 4.0 mm. A drop was formed from 10 μL of 50 μM metal substitution cytochromes c solution (dissolved in 10 mM Tris-HCl (pH 8.0)) on the ITO electrode 22 in the irradiation region 23, which was left for two days at 4 deg C. Accordingly, the protein immobilized electrode was formed.

The protein immobilized electrode was soaked in 27 mL of 10 mM sodium phosphate buffer solution (pH 7.0) containing 0.25 mM potassium ferrocyanide, platinum mesh was used as a counter electrode, a silver/silver chloride electrode was used as a reference electrode, the photocurrent measurement apparatus illustrated in FIG. 4 of Patent document 2 was used, electric potential to the silver/silver chloride electrode was 120 mV, and thereby photocurrent action spectrum in wavelengths from 380 to 600 nm was measured. In the measurement, standby time was 900 sec, measurement time was 60 sec, current range was 10 nA, filter frequency was 30 Hz, and time resolution was 50 mS. For the four types of metal substitution cytochromes c, five electrodes were respectively formed and the measurement was performed.

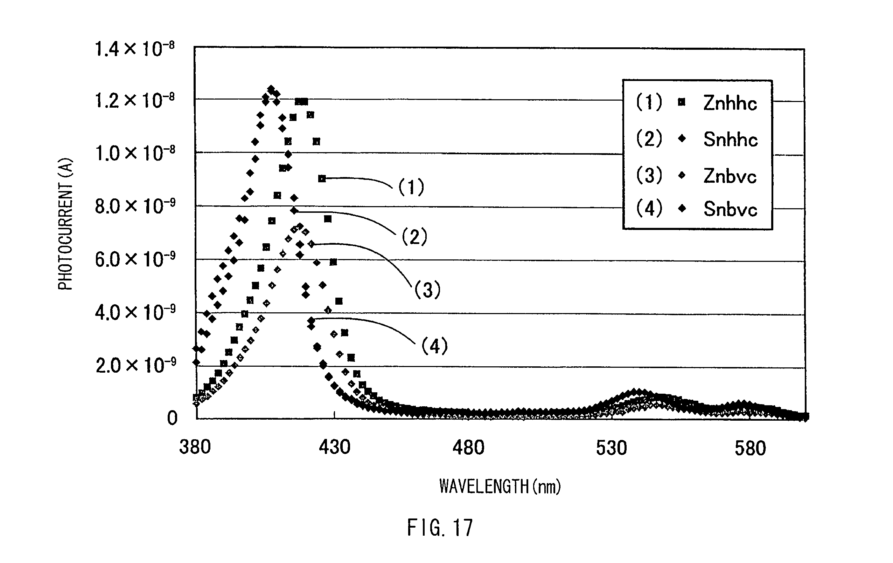

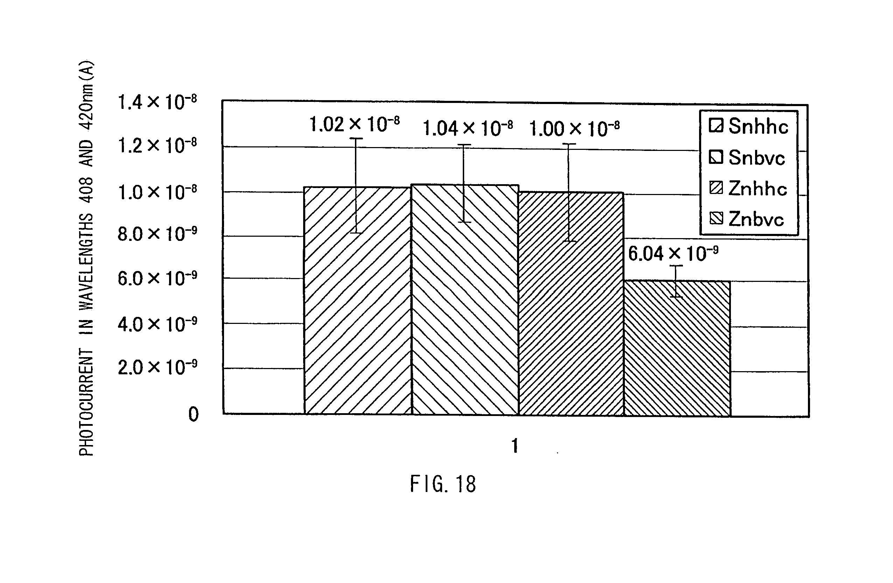

Obtained photocurrent action spectrums are illustrated in FIG. 17. As in the solution absorption spectrum, the spectrum maximum of the photocurrent action spectrums was shown in wavelengths 408, 540, and 578 nm. Based on FIG. 17, the intensity ratio between Soret band (408 nm) and Q band (540 nm) is 10:1. Thus, as in the zinc-substituted horse heart cytochrome c, the photocurrent generation mechanism of the tin-substituted horse heart cytochrome c and the tin-substituted bovine heart cytochrome c is regarded as hole transfer type (Nonpatent document 4). Photocurrent value average value graph in Soret band (the number of samples: 5) is illustrated in FIG. 18. From FIG. 18, it was found that both the tin-substituted horse heart cytochrome c and the tin-substituted bovine heart cytochrome c generated photocurrent (10 nm) similar to that of the zinc-substituted horse heart cytochrome c.

<Fluorescence quantum efficiency of the metal substitution cytochromes c>

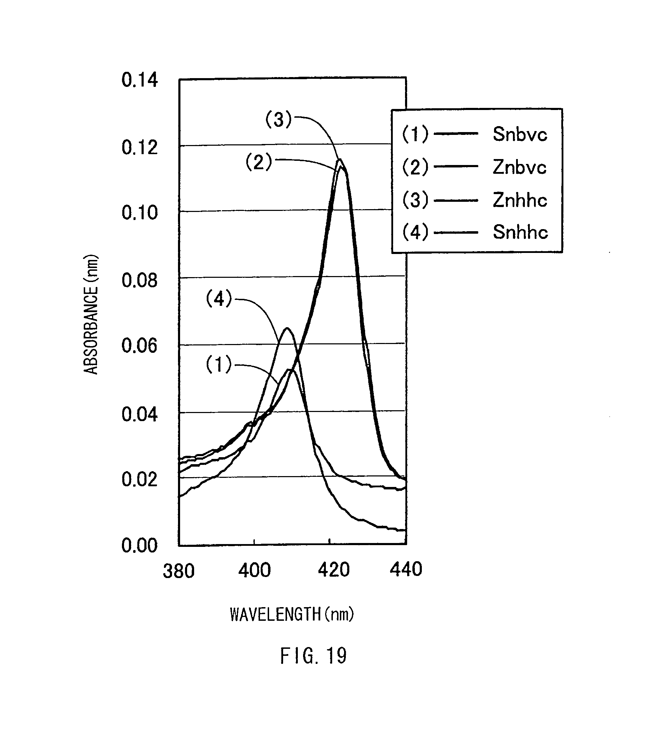

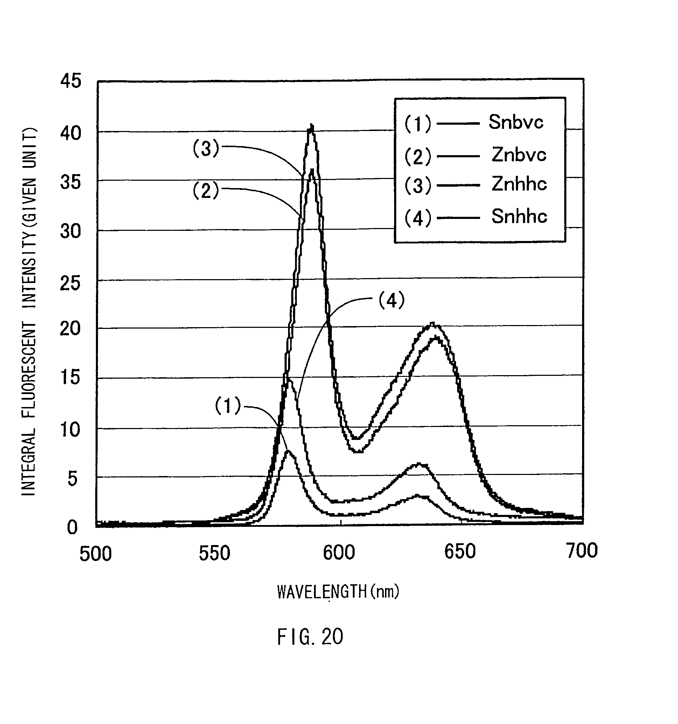

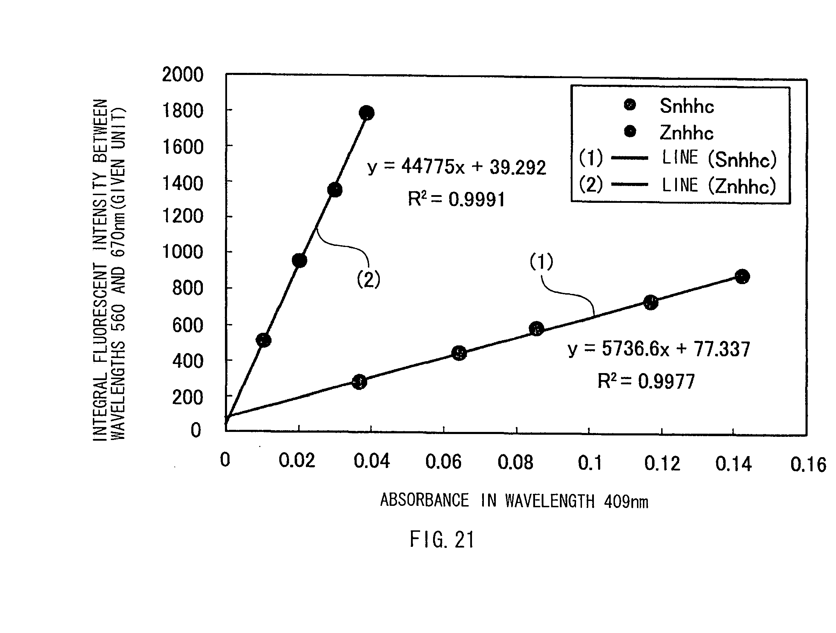

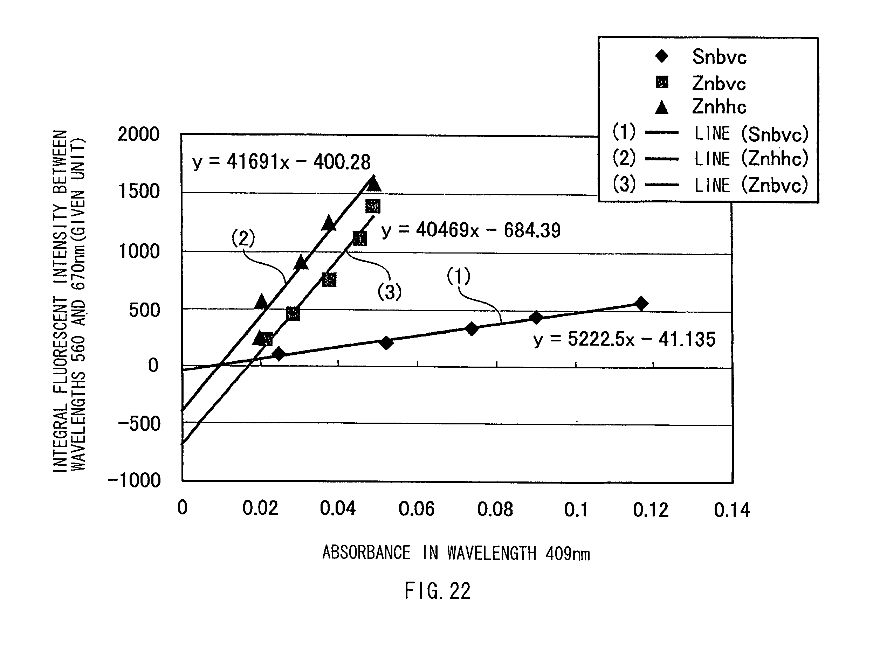

Diluent solutions with different concentrations of the metal substitution cytochrome c were prepared. Ultraviolet visible absorption spectrums in wavelengths from 380 to 440 nm both inclusive and fluorescent spectrums in wavelengths from 500 to 700 nm both inclusive (excitation wavelength: 409 nm) were measured. The results thereof are illustrated in FIG. 19 and FIG. 20. As illustrated in FIG. 21 and FIG 22, straight-line approximate curves were illustrated by plotting respective data based on absorbance in wavelength 409 nm as the horizontal axis (x axis) and integral fluorescent intensity in the range from wavelengths 560 to 670 nm both inclusive as the vertical axis (y axis). Slopes of the obtained straight lines are fluorescence quantum yield. In the fluorescent spectrums illustrated in FIG. 20, an area in the range from wavelengths 560 to 670 nm both inclusive was regarded as integral fluorescent intensity (given unit (a.u.)). The slope of the straight line of the zinc-substituted horse heart cytochrome c, that is, relative fluorescence quantum yield Φ of the respective metal substitution cytochromes c where the fluorescence quantum yield was 1.0 was calculated. The results are illustrated in Table 3. As evidenced by Table 3, the fluorescent intensity of the tin-substituted products is about 1/7 to 1/8 as much as the fluorescent intensity of the zinc-substituted products. The life shortness of excitation electrons in the tin-substituted products may inhibit radical generation at the time of light irradiation and may contribute to stabilization.

[Table 3]

| Protein | Fluorescence quantum yield (Φ) |
|---|---|
| Zinc-substituted horse heart cytochrome c | 1.0 |
| Zinc-substituted bovine heart cytochrome c | 0.97 |
| Tin-substituted horse heart cytochrome c | 0.13 |
| Tin-substituted bovine heart cytochrome c | 0.13 |

As described above, stability to light irradiation of both the tin-substituted horse heart cytochrome c and the tin-substituted bovine heart cytochrome c was significantly higher than stability to light irradiation of the zinc-substituted horse heart cytochrome c and the zinc-substituted bovine heart cytochrome c. Thus, by using the tin-substituted horse heart cytochrome c and the tin-substituted bovine heart cytochrome c, the new protein transparent light-receiving element 1 capable of being stably used for a long time is able to be achieved. The protein transparent light-receiving element 1 is able to be used for a light sensor and an image pickup device. Further, in both the tin-substituted horse heart cytochrome c and the tin-substituted bovine heart cytochrome c, light absorption maximum wavelength is 409 nm, which is close to wavelength 405 nm of laser diodes currently used for optical disc systems available for high density recording. Thus, for example, by using a medium in which the tin-substituted horse heart cytochrome c or the tin-substituted bovine heart cytochrome c is bedded on a substrate instead of an optical disc, a new memory is able to be achieved. Further, since the diameter of the tin-substituted horse heart cytochrome c and the tin-substituted bovine heart cytochrome c is significantly small, about 2 nm, the number of devices capable of being mounted per unit area of a substrate is able to be significantly increased compared to that in the past. Therefore, a high definition light sensor, a high definition image pickup device and the like are able to be achieved, or a large capacity memory is able to be achieved.

[Manufacturing method of multilayer transparent light-receiving device]

A description will be given of an example of a manufacturing method of the multilayer transparent light-receiving device.

First, the transparent electrode 12 formed on the transparent substrate 11 is soaked in a solution containing an electron transfer protein and a buffer solution, and thereby the electron transfer protein is immobilized onto the transparent electrode 12. Accordingly, the protein immobilized electrode in which the electron transfer protein layer 13 is formed on the transparent electrode 12 is formed.

Next, by using the protein immobilized electrode and the transparent counter electrode 15, the protein transparent light-receiving element 1 illustrated in, for example, FIG. 3, FIG. 4, or FIG. 5 is manufactured.

After that, the necessary number of the protein transparent light-receiving devices 1 are laminated. At this time, the respective protein light-receiving devices 1 are bonded to each other by a transparent adhesive or the like according to needs.

[Operation of multilayer transparent light-receiving device]

When monochromatic light of wavelength (for example, about 409 m) corresponding to the electron transfer protein of the electron transfer protein layer 13 or light including the wavelength component enters the electron transfer protein layer 13 of the respective protein transparent light-receiving devices 1 of the multilayer transparent light-receiving device, electrons are generated from the electron transfer protein of the electron transfer protein layer 13 due to light excitation, and the electrons are moved to the transparent electrode 12 by electron transfer. In the result, a photocurrent is extracted outside from the transparent electrode 12 and the transparent counter electrode 15.

As described above, according to the second embodiment, the electron transfer protein layer 13 composed of the tin-substituted horse heart cytochrome c or the tin-substituted bovine heart cytochrome c having high light irradiation stability is immobilized onto the transparent electrode 12. Thus, even if light is irradiated for a ling time, the electron transfer protein layer 13 is not deteriorated, and the new protein transparent light-receiving element 1 capable of being used stably for a long time, that is, the multilayer transparent light-receiving device is able to be achieved.

In the same manner as the multilayer transparent light-receiving device according to the first embodiment, the multilayer transparent light-receiving device is able to be used for various apparatus, devices and the like that use photoelectric conversion. Specifically, for example, the multilayer transparent light-receiving device is able to be used for an electronic device having a light receiving section and the like.

For example, as will be described later, a camera capable of concurrently focusing on a plurality of objects located in a position different from each other by using one lens is able to be achieved. Further, by using the multilayer transparent light-receiving device, multifocusing and high-speed focusing with the use of a single lens are enabled. Further, in the case where the multilayer transparent light-receiving device is used as a light-receiving device of an optical disc system using a multilayer optical disc or an optical recording reproduction system using a holographic recording medium, parallel readout of the multilayer optical disc and readout of the holographic recording medium are able to be easily performed.

<3. Third embodiment>

[Multilayer transparent light-receiving device]

A multilayer transparent light-receiving device according to a third embodiment has a structure similar to that of the multilayer transparent light-receiving device according to the first embodiment, except that a new electron transfer protein is used as an electron transfer protein of the electron transfer protein layer 13 of the protein transparent light-receiving element 1.

The new electron transfer protein is composed of metal substitution cytochrome c obtained by substituting iron as a central metal of heme of mammal-derived cytochrome c with a metal other than zinc and tin whose fluorescent excitation life $\tau$ is $5.0 \times 10^{-11}$ s$<\tau<8.0 \times 10$-10 s, or a protein that is composed of an amino-acid sequence obtained by losing, substituting, or adding one or several amino acids in an amino-acid sequence of the mammal-derived cytochrome c and that contains a metal other than zinc and tin whose fluorescent excitation life $\tau$ is $5.0 \times 10^{-11}$ s$<\tau<8.0 \times 10^{-10}$ s. Examples of the mammal-derived cytochrome c include the horse heart cytochrome c and the bovine heart cytochrome c. These new electron transfer proteins have significantly high stability to light irradiation, and are able to retain photoelectric conversion function for a long time.

<Metal substitution cytochrome c>

A description will be given of metal substitution horse heart cytochrome c and metal substitution bovine heart cytochrome c obtained by substituting iron as a central metal of heme of the horse heart cytochrome c and the bovine heart cytochrome c with a metal other than tin and zinc.

Examples of metals used for the metal substitution horse heart cytochrome c and the metal substitution bovine heart cytochrome c are illustrated in Table 4. It has been known that porphyrins containing these metals as a central metal generate fluorescence (Nonpatent document 5). In Table 4, the numerical values described under the respective atomic symbols indicate each phosphorescence life measured for metal octaethylporphyrins.

[Table 4]

| | Be(II) 12 ms | | | | | | | |
|---|---|---|---|---|---|---|---|---|
| Na(I)~50 ms | Mg(II) 130 ms | | | | | | | |
| K(I) ~50 ms | Ca(II) 63 ms | Sc(III) 400 ms | Ti(IV) 41 ms | | | Al(III) 115 ms | Si (IV) 95 ms | |
| | Sr(II) 7 ms | Y(III) 55 ms | Zr(IV) 65 ms | Nb(V) 14.5 ms | Zn(II) 76 ms | | Ge(IV) 42 ms | As(V) 86 ms |

(continued)

| | Ba(II) 8 ms | Lu(III) 7.8 ms | Hf(IV) 8.4 ms | Ta(V) 2.5 ms | Cd(II) 7 ms | | Sn(IV) 30 ms | Sb(V) 26 ms |
|---|---|---|---|---|---|---|---|---|
| | | | Th(IV) 0.43 ms | | | | Pb(IV) 2.8ms | |

As illustrated in Table 4, the phosphorescence life of tin (Sn) porphyrin is 30 ms. Metal porphyrins whose phosphorescence life is equal to or shorter than the phosphorescence life of tin (Sn) porphyrin possibly do not damage a protein or a porphyrin ring section due to light irradiation. As illustrated in Table 4, such metals include beryllium (Be), strontium (Sr), niobium (Nb), barium (Ba), lutetium (Lu), hafnium (Hf), tantalum (Ta), cadmium (Cd), antimony (Sb), thorium (Th), and lead (Pb).

The iron as a central metal of heme of the horse heart cytochrome c and the bovine heart cytochrome c is substituted with these metals. For such substitution, a method similar to that described in the second embodiment is able to be used.

In the metal substitution horse heart cytochrome c and the metal substitution bovine heart cytochrome c obtained as above, it is stable to light irradiation as in the tin-substituted horse heart cytochrome c and the tin-substituted bovine heart cytochrome c, and light degradation is hardly generated.

Here, a description will be given of fluorescent excitation life range needed for the metal substitution horse heart cytochrome c and the metal substitution bovine heart cytochrome c.

Intramolecular hole transfer rate of the zinc-substituted horse heart cytochrome c (Nonpatent document 4) is as follows. When molecular orbital numbers in accordance with Nonpatent document 4 are used as a molecular orbital (MO) number, the intramolecular hole transfer rate of the zinc-substituted horse heart cytochrome c is $1.5 \times 10^{11}s^{-1}$ in transition between MO3272 and MO3271, and is $2.0 \times 10^{10}s^{-1}$ in transition between MO3268 and MO3270. In this case, the lower limit of the intramolecular hole transfer rate is set to the latter value, $2.0 \times 10^{10}s^{-1}$.

Fluorescent excitation life of the tin-substituted horse heart cytochrome c (Nonpatent document 3) is $8.0 \times 10^{-10}s$. Fluorescent excitation life of the zinc-substituted horse heart cytochrome c is $3.2 \times 10^{-10}s$.

The number of intramolecular hole transfers one electron excitation of the tin-substituted horse heart cytochrome c is $(1.5 \times 10^{11}s^{-1})$ x $(8.0 \times 10^{-10}s)$=120 in transition between MO3272 and MO3271, and $(2.0 \times 10^{10}s^{-1})$ x $(8.0 \times 10^{-10}s)$ =16 in transition between MO3268 and MO3270. In this case, the lower limit of the number of intramolecular hole transfers in one electron excitation is set to the latter value, 16.

In this case, fluorescent excitation life needed for generating at least one hole transfer is $8.0 \times 10^{-10}s/ 16=5.0 \times 10^{-11}$ s.

Accordingly, fluorescent excitation life ($\tau$) range of the metal substitution horse heart cytochrome c and the metal substitution bovine heart cytochrome c that does not damage a protein portion or porphyrin due to light irradiation and that is needed for generating hole transfer is $5.0 \times 10^{-11}s$ (fluorescent excitation life needed for generating at least one hole transfer)$<\tau<8.0 \times 10^{-10}s$ (fluorescent excitation life of the tin-substituted horse heart cytochrome c).

According to the third embodiment, the metal substitution horse heart cytochrome c or the metal substitution bovine heart cytochrome c is used as an electron transfer protein of the electron transfer protein layer 13 of the protein transparent light-receiving element 1. Thereby, advantages similar to those of the multilayer transparent light-receiving device according to the second embodiment using the tin-substituted horse heart cytochrome c and the tin-substituted bovine heart cytochrome c are able to be obtained.

<4. Fourth embodiment>

[Multilayer transparent light-receiving device]

A multilayer transparent light-receiving device according to a fourth embodiment has a structure similar to that of the multilayer transparent light-receiving device according to the first embodiment, except that a solid protein layer composed of an electron transfer protein as the electron transfer protein layer 13 of the protein transparent light-receiving element 1 is used.

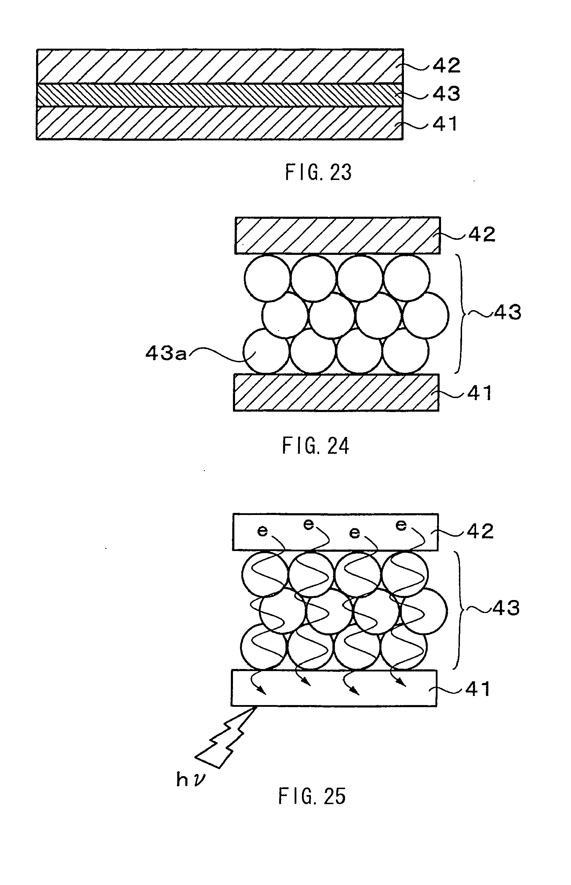

FIG. 23 illustrates a non-wetted all solid protein transparent light-receiving device used as the protein transparent light-receiving element 1. In the non-wetted all solid protein transparent light-receiving device, a solid protein layer is used. Here, the solid protein layer means a lamellar solid composed of protein assembly without containing liquid such as water. Further, "non-wetted" of the non-wetted all solid protein transparent light-receiving device means that the device is used in a state that inside and outside of the protein transparent light-receiving device are not contacted with liquid such as water. Further, "all solid" of the non-wetted all solid protein transparent light-receiving device means that all regions of the device do not contain liquid such as water.

As illustrated in FIG. 23, the non-wetted all solid protein transparent light-receiving device has a structure in which a solid protein layer 43 composed of the electron transfer protein is sandwiched between a transparent electrode 41 and a transparent electrode 42. The solid protein layer 43 is immobilized onto the transparent electrodes 41 and 42. The solid protein layer 43 is typically immobilized onto the transparent electrodes 41 and 42 directly. However, according to needs, an intermediate layer not containing liquid such as water may be provided between the solid protein layer 43 and the transparent electrodes 41 and 42. Liquid such as water is not contained in the solid protein layer 43. The solid protein layer 43 is composed of a protein monomolecular film or a protein multimolecular film.

An example of a structure in the case that the solid protein layer 43 is composed of a multimolecular film is illustrated in FIG. 24. As illustrated in FIG. 24, the solid protein layer 43 is obtained by laminating n layers (n is an integer number equal to or greater than 2) of monomolecular films that are formed from two dimensional assembly of electron transfer proteins 43a composed of, for example, the tin-substituted horse heart cytochrome c, the tin-substituted bovine heart cytochrome c, the zinc-substituted horse heart cytochrome c or the like. FIG. 24 illustrates a case of n=3.

As a material of the transparent electrodes 41 and 42, a material similar to that of the transparent electrode 12 is able to be used. Specifically, the transparent electrodes 41 and 42 are made of a conductive material transparent to light used for light excitation such as ITO, FTO, and NESA glass, an extremely thin Au film capable of transmitting light or the like.

Next, a description will be given of a manufacturing method of the non-wetted all solid protein transparent light-receiving device.

First, a solution containing the electron transfer proteins 43a, specifically, a protein solution obtained by dissolving the electron transfer proteins 43a in a buffer solution containing water is attached onto one of the transparent electrodes 41 and 42, for example, onto the transparent electrode 41 by liquid drop method, spin coat method, dip method, spray method or the like. Next, the resultant obtained by attaching the protein solution onto the transparent electrode 41 is retained at room temperature or at temperature lower than room temperature. Thereby, the electron transfer proteins 43a in the attached protein solution are immobilized onto the transparent electrode 41.

Next, the resultant obtained by immobilizing the electron transfer proteins 43a in the protein solution onto the transparent electrode 41 is heated up to temperature lower than denaturation temperature of the electron transfer protein 43a and dried. Thereby, the liquid contained in the protein solution is all evaporated and removed.

Accordingly, only the electron transfer proteins 43a are immobilized onto the transparent electrode 41, and thereby the solid protein layer 43 is formed. The thickness of the solid protein layer 43 is able to be easily controlled by the amount of the protein solution attached onto the transparent electrode 41, the concentration of the protein solution and the like. Next, the transparent electrode 42 is formed on the solid protein layer 43. The transparent electrode 42 is able to be formed by depositing a conductive material by sputtering method, vacuum evaporation method or the like.

Accordingly, the intended non-wetted all solid protein transparent light-receiving device is manufactured.

Next, a description will be given of operation of the non-wetted all solid protein transparent light-receiving device.

A voltage (a bias voltage) is applied between the transparent electrode 41 and the transparent electrode 42 of the non-wetted all solid protein transparent light-receiving device so that the transparent electrode 42 side has lower electric potential. In the case where light does not enter the solid protein layer 43 of the non-wetted all solid protein transparent light-receiving device, the solid protein layer 43 is insulative and a current is not flown between the transparent electrode 41 and the transparent electrode 42. Such a state is off-state of the non-wetted all solid protein transparent light-receiving device. Meanwhile, as illustrated in FIG. 25, for example, in the case where light (h$\upsilon$) enters the solid protein layer 43 through the transparent electrode 41, the electron transfer proteins 43a composing the solid protein layer 43 are light-excited, and as a result, the solid protein layer 43 becomes conductive. Accordingly, electrons (e) are flown through the solid protein layer 43 from the transparent electrode 42 to the transparent electrode 41, and a photocurrent is flown between the transparent electrode 41 and the transparent electrode 42. Such a state is on-state of the non-wetted all solid protein transparent light-receiving device. As described above, the solid protein layer 43 behaves as a photoconductor, and enables on/off operation according to presence of light entrance to the non-wetted all solid protein transparent light-receiving device.

<Example>

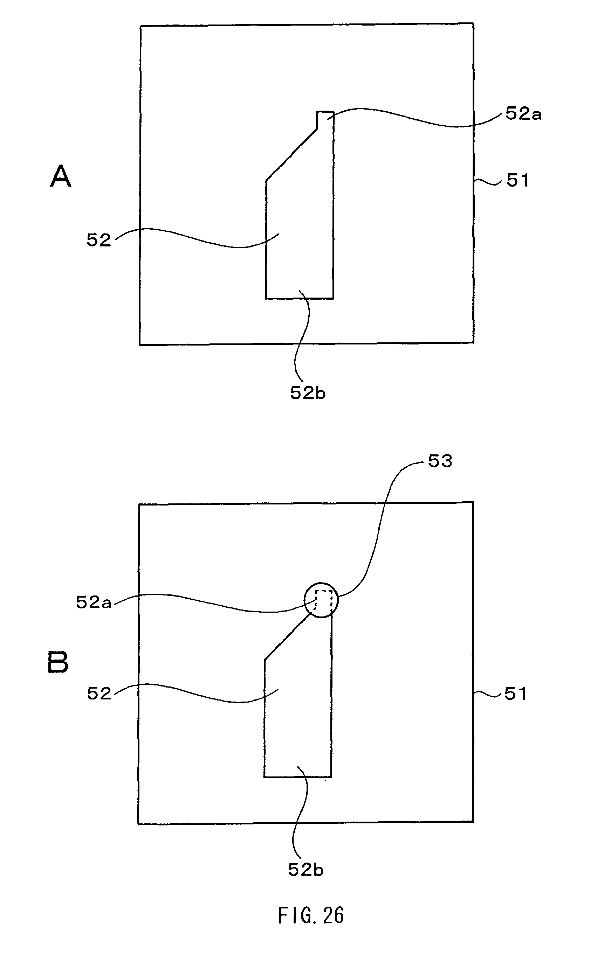

As illustrated in FIG. 26A, an ITO electrode 52 in a given shape was formed as the transparent electrode 41 on the glass substrate 51. The thickness of the ITO electrode 52 was 100 nm, and the area thereof was 1 mm$^2$. The ITO electrode 52 was a working electrode.

Protein solutions (200 $\mu$M) obtained by respectively dissolving the tin-substituted horse heart cytochrome c, the tin-substituted bovine heart cytochrome c, and the zinc-substituted horse heart cytochrome c in concentrated form in Tris-HCl buffer solution (pH8.0) were prepared.

Next, as illustrated in FIG. 26B, 10 $\mu$L of the protein solutions prepared as described above were dropped onto one end portion 52a of the ITO electrode 52, and thereby a protein droplet 53 was attached onto the ITO electrode 52.

Next, the resultant was left for 2 hours at room temperature, or for a day and a night at 4 deg C, and thereby the tin-substituted horse heart cytochrome c, the tin-substituted bovine heart cytochrome c, or the zinc-substituted horse heart cytochrome c in the protein droplet 53 was immobilized onto the ITO electrode 52.

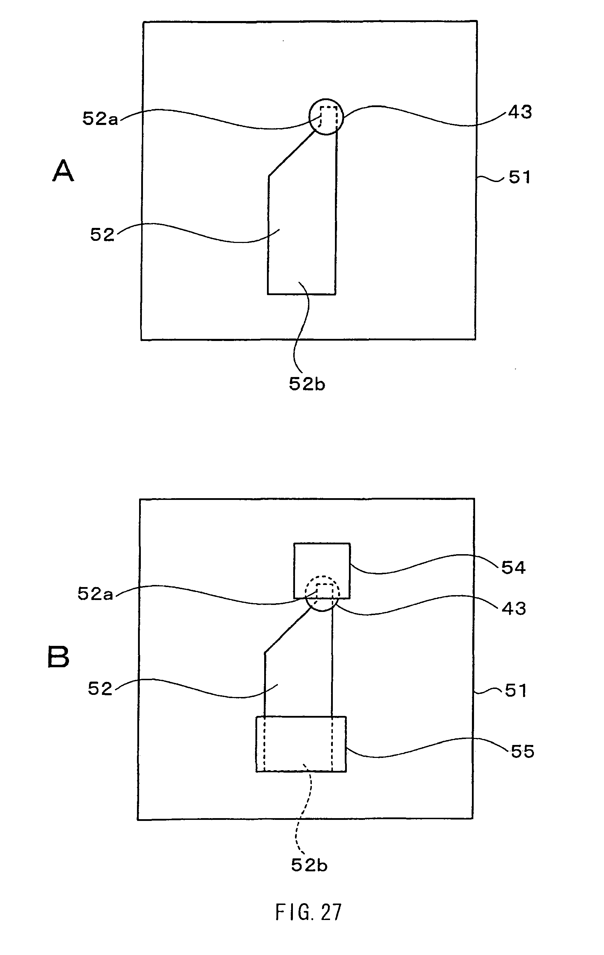

Next, the samples were put into a drier machine retained at 30 to 40 deg C both inclusive, and were dried for 30 to 60 minutes both inclusive. By such drying process, liquid such as water contained in the protein droplet 53 was vaporized and removed. In the result, only the tin-substituted horse heart cytochrome c, the tin-substituted bovine heart cytochrome c, or the zinc-substituted horse heart cytochrome c was left on the ITO electrode 52, and the solid protein layer 43 was formed as illustrated in FIG. 27A. The thickness of the solid protein layer 43 was about 1 μm.

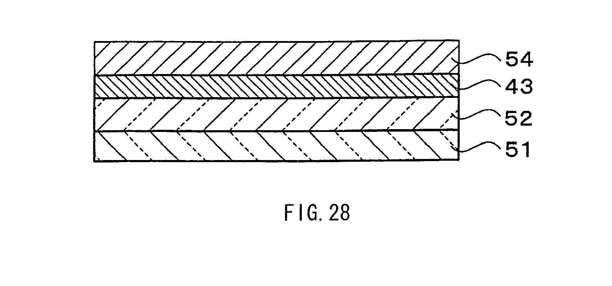

Next, as illustrated in FIG. 27B, a transparent electrode 54 was formed to overlap the solid protein layer 43, and a transparent electrode 55 was formed to overlap the other end portion 52b of the ITO electrode 52. The transparent electrode 55 was used as a counter electrode and a working electrode. The transparent electrodes 54 and 55 were formed from a Au film or an Al film. The thickness of the Au film was 20 nm, and the thickness of the Al film was 50 nm. The transparent electrodes 54 and 55 were able to be formed by masking sections other than regions in which the transparent electrodes 54 and 55 were formed and depositing a transparent electrode material by sputtering method or vacuum evaporation method. The planar shape of the transparent electrodes 54 and 55 was a rectangle or a square. Accordingly, the non-wetted all solid protein transparent light-receiving device was manufactured. The cross sectional structure of the non-wetted all solid protein transparent light-receiving device is illustrated in FIG. 28.

Many non-wetted all solid protein transparent light-receiving devices were manufactured as described above, and resistance between the transparent electrodes 54 and 55 was measured in the air. In the result, the resistance was distributed in a wide range from 1 kΩ to 30 MΩ both inclusive. The reason why the resistance between the transparent electrodes 54 and 55 was distributed in the wide range as described above was as follows. That is, the thickness of the solid protein layer 43 varied according to each device, or each type of the electron transfer proteins 43a composing the solid protein layer 43 varied according to each device.

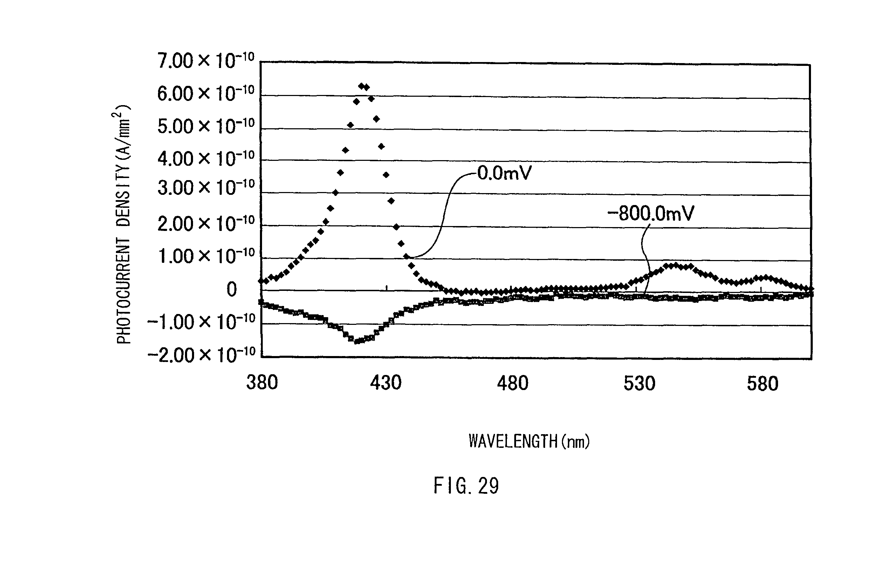

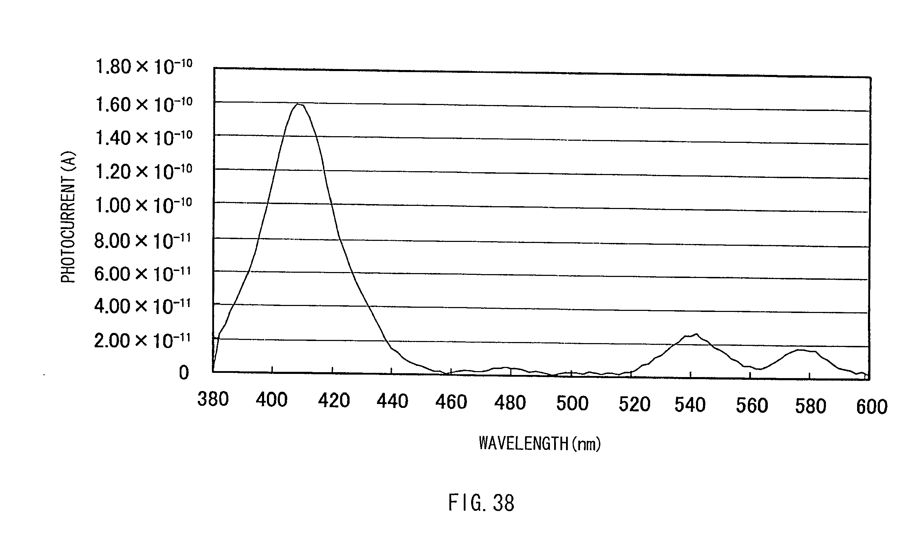

Photocurrent action spectrum of the non-wetted all solid protein transparent light-receiving device was measured. As the electron transfer proteins 43a composing the solid protein layer 43, the tin-substituted horse heart cytochrome c and the zinc-substituted horse heart cytochrome c were used. Measurement was performed by connecting a working electrode of a potentiostat to the transparent electrode 54 connected to the ITO electrode 52, and connecting a counter electrode and a reference electrode to the transparent electrode 55. The transparent electrodes 54 and 55 were made of the Au film being 20 nm thick. Measurement result of action spectrum under potential of 0 mV and -800 mV in the case where the zinc-substituted horse heart cytochrome c was used as the electron transfer proteins 43a composing the solid protein layer 43 is illustrated in FIG. 29. Further, measurement result of action spectrum under potential of 0 mV in the case where the tin-substituted bovine heart cytochrome c was used as the electron transfer proteins 43a composing the solid protein layer 43 is illustrated in FIG. 38. As illustrated in FIG. 29 and FIG. 38, both in the case where the zinc-substituted horse heart cytochrome c was used as the electron transfer proteins 43a composing the solid protein layer 43 and in the case where tin-substituted bovine heart cytochrome c was used as the electron transfer proteins 43a composing the solid protein layer 43, action spectrum was able to be observed. In particular, as illustrated in FIG. 29, in the case where the zinc-substituted horse heart cytochrome c was used as the electron transfer proteins 43a composing the solid protein layer 43, action spectrums in both positive and negative directions were able to be observed. Further, as illustrated in FIG. 29, action spectrum was able to be measured even under overvoltage -800 mV, which was a new finding and was a significantly remarkable result.

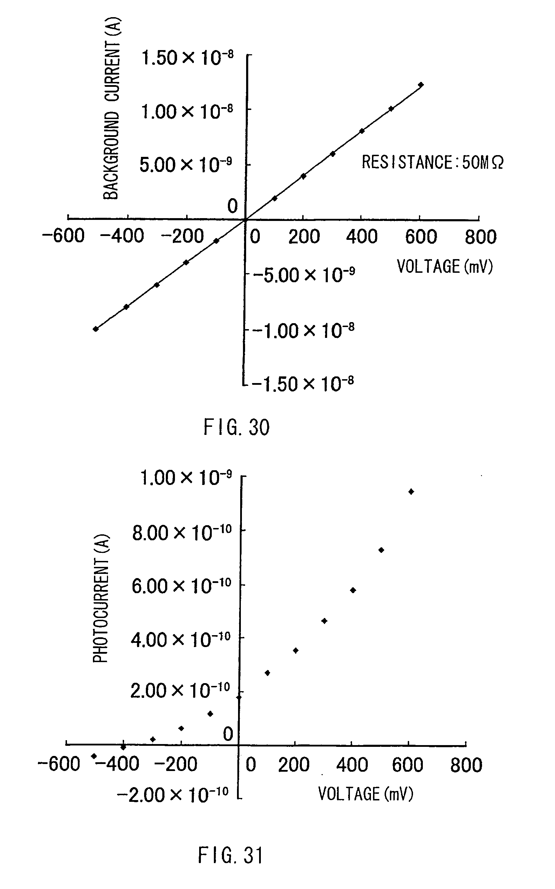

FIG. 30 illustrates measurement result of a background current (current flown at the time of light off) at each voltage in the case where a voltage (a bias voltage) was applied between the transparent electrodes 54 and 55 of the non-wetted all solid protein transparent light-receiving device using the zinc-substituted horse heart cytochrome c as the electron transfer proteins 43a composing the solid protein layer 43. As illustrated in FIG. 30, a curve indicating relation between a voltage and a background current is the straight line, which means conductivity of the solid protein layer 43 resembles that of semiconductor. From the slope of the straight line, it is found that resistance between the transparent electrodes 54 and 55 is about 50 MΩ.

FIG. 31 illustrates measurement result of a photocurrent (current flown at the time of light on) at each voltage in the case where a voltage was applied between the transparent electrodes 54 and 55 of the non-wetted all solid protein transparent light-receiving device using the zinc-substituted horse heart cytochrome c as the electron transfer proteins 43a composing the solid protein layer 43. As illustrated in FIG. 31, a curve indicating relation between a voltage and a photocurrent is also the approximately straight line, which means that the solid protein layer 43 functioned as a photoconductor.

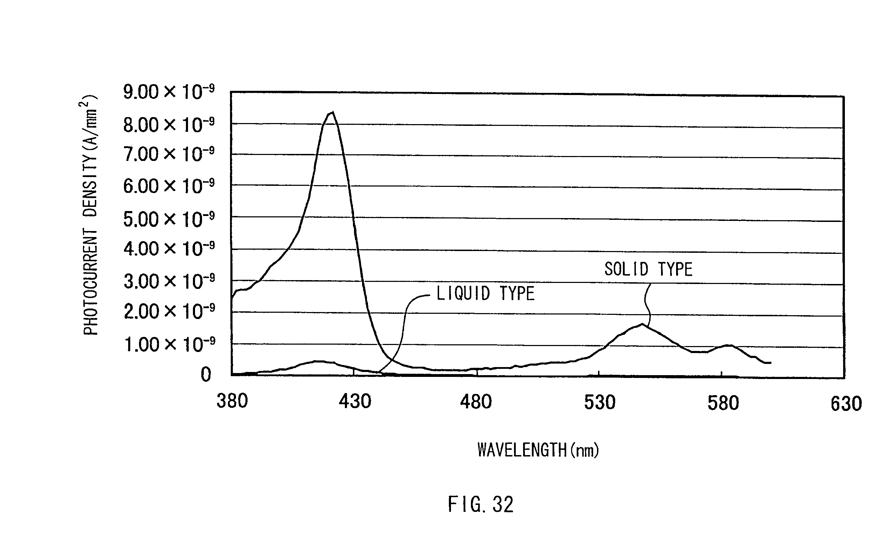

FIG. 32 illustrates measurement result of photocurrent action spectrum of the non-wetted all solid protein transparent light-receiving device using the zinc-substituted horse heart cytochrome c as the electron transfer proteins 43a composing the solid protein layer 43 and a liquid type protein transparent light-receiving device formed by the after-mentioned method. In FIG. 32 and FIG. 33 to FIG. 35 described below, the foregoing non-wetted all solid protein transparent light-receiving device will be abbreviated to "solid type," and the liquid type protein transparent light-receiving device will be abbreviated to "liquid type."

The liquid type protein transparent light-receiving device was formed as described below. First, a given region on the surface of an ITO film formed on a glass substrate was masked with the use of a tape or a resin. Next, a portion of the ITO film not masked was removed by wet etching for 90 sec by using 12M HCl (50 deg C). Next, after the glass substrate was washed with water, the mask was removed, and the resultant was dried in airflow. Next, the glass substrate was provided with ultrasonic treatment for 30 minutes in 1% Alconox (registered trademark) aqueous solution, was subsequently provided with ultrasonic treatment for 15 minutes in isopropanol, and was provided with ultrasonic treatment for 15 minutes in water twice. Next, after the glass substrate was soaked in 0.01 M NaOH for 3 minutes, the resultant was dried in airflow or nitrogen flow. After that, the glass substrate was provided with ultraviolet (UV)-ozone surface treatment for 15 minutes at about 60 deg C. Accordingly, the ITO electrode was formed. The ITO electrode was a working electrode. Next, in the first method, the ITO electrode formed as described above was rinsed with a protein solution (50 $\mu$M) obtained by dissolving the zinc-substituted horse cytochromes c in Tris-HCl buffer solution (pH 8.0). Next, after the ITO electrode rinsed with the protein solution as above was retained for a night at 4 deg C, the resultant was rinsed with water and was dried in airflow or nitrogen flow. In the second method, the ITO electrode formed as described above was rinsed with a protein solution (50 $\mu$M) obtained by dissolving the zinc-substituted horse cytochromes c in Tris-HCl buffer solution (pH 8.0). Otherwise, the ITO electrode formed as described above was rinsed with a protein solution (5 $\mu$M) obtained by dissolving the zinc-substituted horse cytochromes c in sodium phosphate buffer solution (pH 7.0). Next, the ITO electrode rinsed with the protein solution as above was dried in vacuum. After that, the ITO electrode was rinsed with water and was dried in airflow or nitrogen flow. As described above, a protein immobilized electrode in which the protein was immobilized onto the ITO electrode was formed. Next, the protein side of the protein immobilized electrode was placed opposite a clean ITO electrode separately formed as a counter electrode with a given distance in between. Outer circumferential sections of the protein immobilized electrode and the ITO electrode were sealed with a resin. In the ITO electrode as the counter electrode, a pinhole that communicates with a space between the protein immobilized electrode and the ITO electrode was formed as an air hole. Next, the resultant obtained by sealing the circumferential sections of the protein immobilized electrode and the ITO electrode with the resin was soaked in an electrolyte solution contained in a container. As the electrolyte solution, a solution obtained by dissolving 0.25 mM potassium ferrocyanide in 10 mM sodium phosphate buffer solution (pH 7.0) was used. Next, the container was retained in vacuum, and air in the space between the protein immobilized electrode and the ITO electrode was discharged outside from the foregoing pinhole. Next, the container was returned back under atmosphere pressure, and the space between the protein immobilized electrode and the ITO electrode was filled with the electrolyte solution. After that, the foregoing pinhole was sealed with a resin. Accordingly, the liquid type protein transparent light-receiving device was formed.

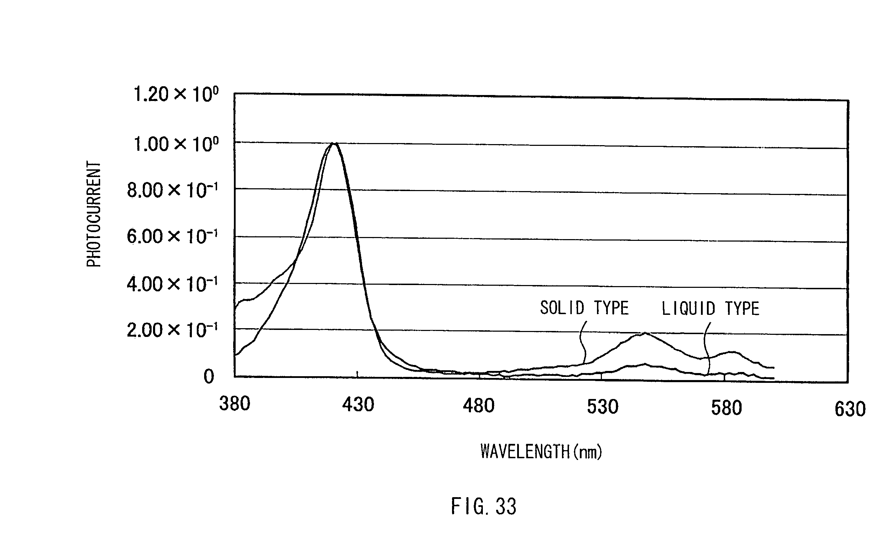

FIG. 33 is a graph obtained by normalizing the spectrums of the non-wetted all solid protein transparent light-receiving device and the liquid type protein transparent light-receiving device illustrated in FIG. 32 so that the photocurrent density of the peak in the vicinity of wavelength 420 nm is 1. As illustrated in FIG. 32, though the respective photocurrent densities of both spectrums differ from each other. However, each peak wavelength in Soret band in the vicinity of wavelength 423 nm and Q band in the vicinity of wavelength 550 nm and wavelength 583 nm is identical. Thus, it is found that in both cases, a photocurrent derived from the zinc-substituted horse cytochromes c was obtained. The inventors were the first finders who found the fact that the photocurrent derived from the zinc-substituted horse cytochromes c was obtained in the non-wetted all solid protein transparent light-receiving device using the solid protein layer 43 composed of the zinc-substituted horse heart cytochrome c. That finding is an amazing result that reverses existing common sense.

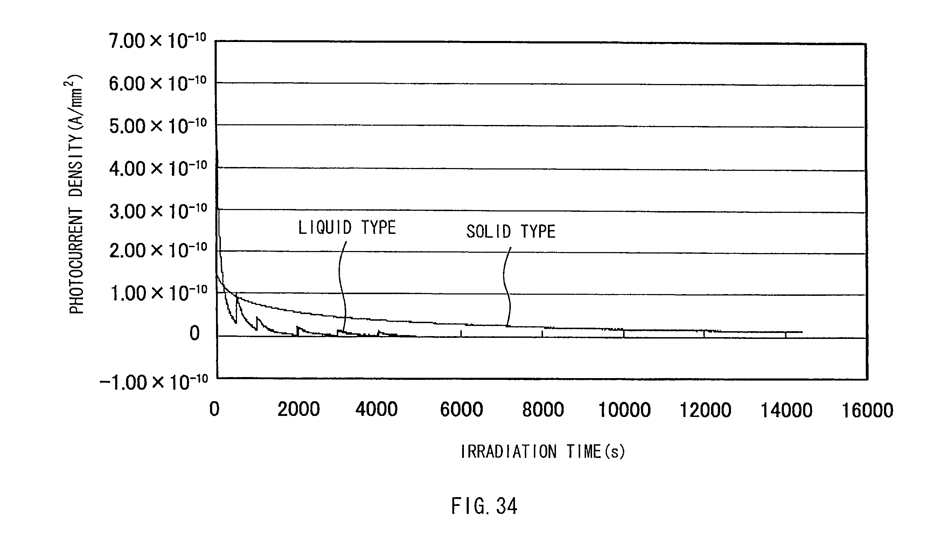

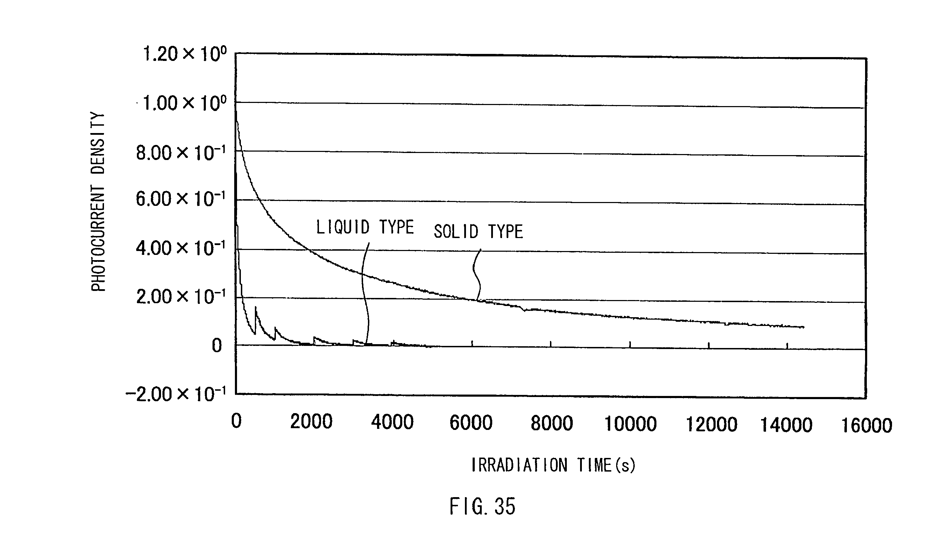

FIG. 34 illustrates measurement results of light degradation curves (curves indicating decrease of photocurrent density to light irradiation time) for the foregoing non-wetted all solid protein transparent light-receiving device and the foregoing liquid type protein transparent light-receiving device. The measurement was performed by measuring photocurrent density while irradiating the non-wetted all solid protein transparent light-receiving device and the liquid type protein transparent light-receiving device with laser light in wavelength 405.5 nm at an intensity of 0.2 mW/mm$^2$. The irradiation intensity of the laser light was high, 0.2 mW/mm$^2$ in order to increase light degradation rate and shorten test time. FIG. 35 is a graph obtained by normalizing the light degradation curves of the non-wetted all solid protein transparent light-receiving device and the liquid type protein transparent light-receiving device illustrated in FIG. 34 so that the photocurrent density when the irradiation time is 0 becomes 1.

The light degradation curves illustrated in FIG. 35 were provided with fitting with the use of the following function.

$$f(x) = a \times exp(b \times x) + c \times exp(d \times x)$$

Coefficients a, b, c, and d of the function f(x) are as follows. Numerical values in parentheses of each coefficient indicate 95% confidence interval.

Liquid type protein transparent light-receiving device

$$a=5.204 \times 10^{-9} \ (5.029 \times 10^{-9}, \ 5.378 \times 10^{-9})$$

$$b=-0.00412 \ (-0.00441, \ -0.003831)$$

$$c=1.799 \times 10^{-10} \ (2.062 \times 10^{-11}, \ 3.392 \times 10^{-10})$$

$$d=-0.0004618 \ (-0.000978, \ -2.58 \times 10^{-5})$$

Non-wetted all solid protein transparent light-receiving device

$$a=5.067 \times 10^{-11} \ (4.883 \times 10^{-11}, \ 5.251 \times 10^{-11})$$

$$b=-0.0009805 \ (-0.001036, \ -0.0009249)$$

$$c=4.785 \times 10^{-11} \ (4.58 \times 10^{-11}, \ 4.99 \times 10^{-11})$$

$$d=-0.0001298 \ (-0.0001374, \ -0.0001222)$$

Life t of the non-wetted all solid protein transparent light-receiving device and the liquid type protein transparent light-receiving device is defined as $t=[a/(a+c)](-1/b)+[c/(a+c)](-1/d)$. According to the definition, the life of the liquid type protein transparent light-receiving device is 306 sec, while the life of the non-wetted all solid protein transparent light-receiving device is 4266 sec. Thus, it is found that the life of the non-wetted all solid protein transparent light-receiving device is at least 14 times as much as the life of the liquid type protein transparent light-receiving device.

In addition, in the light degradation curve of the liquid type protein transparent light-receiving device illustrated in FIG. 34, saw-like waveform is shown for the following reason. That is, the measurement needed to be interrupted in order to remove oxygen generated in the electrolyte solution. After operation of removing oxygen, a photocurrent is slightly increased.

Next, a description will be given of results of measuring frequency response of the non-wetted all solid protein transparent light-receiving device and the liquid type protein transparent light-receiving device.

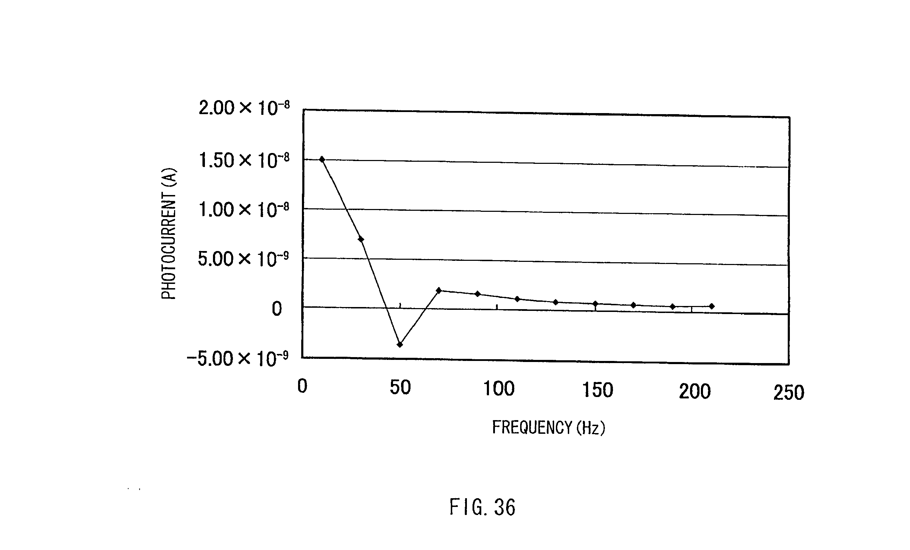

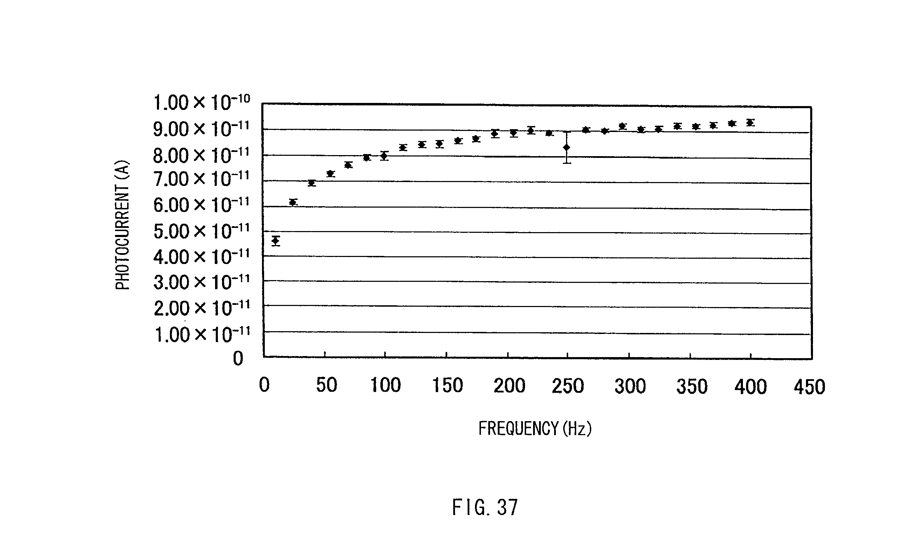

FIG. 36 illustrates measurement result of frequency response of the liquid type protein transparent light-receiving device, and FIG. 37 illustrates measurement result of frequency response of the non-wetted all solid protein transparent light-receiving device. From FIG. 36 and FIG. 37, 3dB bandwidth (frequency at which a photocurrent value became 50% as much as the maximum photocurrent value) of the liquid type protein transparent light-receiving device is lower than 30 Hz, while 3dB bandwidth of the non-wetted all solid protein transparent light-receiving device is 400 Hz or more. Accordingly, it is found that the response rate of the non-wetted all solid protein transparent light-receiving device is at least 13 times as much as the response rate of the liquid type protein transparent light-receiving device.

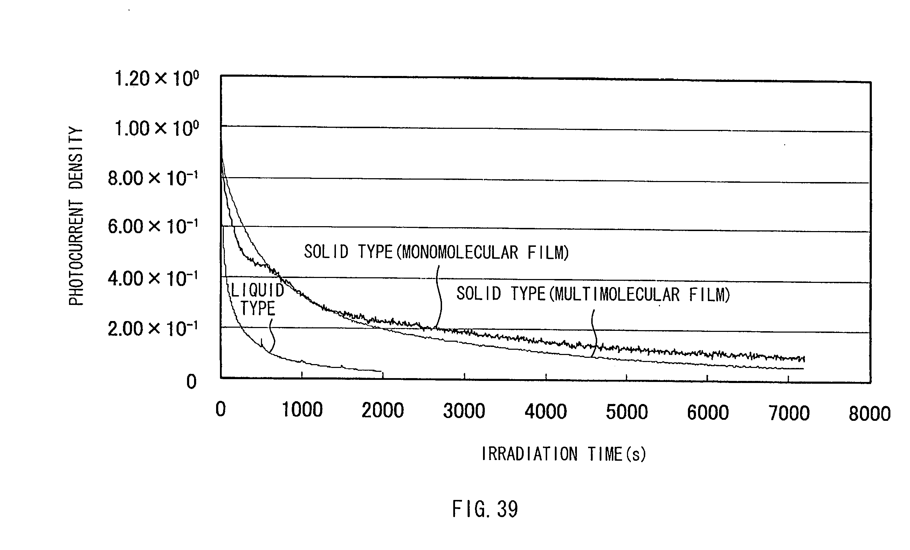

FIG. 39 is a graph obtained by measuring light degradation curves for the non-wetted all solid protein transparent light-receiving device using the tin-substituted bovine heart cytochrome c as the electron transfer proteins 43a composing the solid protein layer 43 and the liquid type protein transparent light-receiving device using the tin-substituted bovine heart cytochrome c and normalizing the light degradation curves so that the photocurrent density when the irradiation time is 0 becomes 1. A formation method of the liquid type protein transparent light-receiving device was similar to the foregoing method, except that the tin-substituted bovine heart cytochrome c was used instead of the zinc-substituted horse heart cytochrome c. As the non-wetted all solid protein transparent light-receiving device, a device having a monomolecular film of the tin-substituted bovine heart cytochrome c and a device having a multimolecular film of the tin-substituted bovine heart cytochrome c were formed. The measurement was performed by measuring photocurrent density while irradiating the non-wetted all solid protein transparent light-receiving device and the liquid type protein transparent light-receiving device with laser light in wavelength 405.5 nm at an intensity of 0.2 mW/mm$^2$. The irradiation

intensity of the laser light was high, 0.2 mW/mm$^2$ in order to increase light degradation rate and shorten test time.

The light degradation curves illustrated in FIG. 39 were provided with fitting with the use of the following function.

$$f(x)=a \times exp(b \times x)+c \times exp(d \times x)$$

Coefficients a, b, c, and d of the function f(x) are as follows.
Liquid type protein transparent light-receiving device

$$a=1.72 \times 10^{-8}$$

$$b=-0.00462$$

$$c=3.51 \times 10^{-9}$$

$$d=-0.000668$$

Non-wetted all solid protein transparent light-receiving device (monomolecular film)

$$a=0.4515$$

$$b=-0.002599$$

$$c=0.3444$$

$$d=-0.0001963$$

Non-wetted all solid protein transparent light-receiving device (multimolecular film)

$$a=0.5992$$

$$b=-0.002991$$

$$c=0.2371$$

$$d=-0.0001513$$

In this case, light degradation average time constant of the non-wetted all solid protein transparent light-receiving device and the liquid type protein transparent light-receiving device was as follows.
Liquid type protein transparent light-receiving device: $2.54 \times 10^2$ sec
Non-wetted all solid protein transparent light-receiving device (monomolecular film) : $2.71 \times 10^3$ sec
Non-wetted all solid protein transparent light-receiving device (multimolecular film) : $2.73 \times 10^3$ sec
As described above, life t of the non-wetted all solid protein transparent light-receiving device and the liquid type protein transparent light-receiving device is defined as t=[a/(a+c)](-1/b)+[c/(a+c)](-1/d). According to the definition, the life of the liquid type protein transparent light-receiving device is 434 sec, while the life of the non-wetted all solid protein transparent light-receiving device (monomolecular film) is 2423 sec, and the life of the non-wetted all solid protein transparent light-receiving device (multimolecular film) is 2113 sec. Thus, it is found that the life of the non-wetted all solid protein transparent light-receiving device is at least about 5 times as much as the life of the liquid type protein transparent light-receiving device.

According to the multilayer transparent light-receiving device according to the fourth embodiment, the following various advantages are able to be obtained. That is, in the non-wetted all solid protein transparent light-receiving device used as the protein transparent light-receiving element 1 composing the multilayer transparent light-receiving device, water does not exist inside the device, and operation is enabled without being contacted with water. Thus, as a light-receiving device replacing the existing light-receiving device using semiconductor, the non-wetted all solid protein transparent light-receiving device is able to be mounted onto an electronic device. Further, in the non-wetted all solid protein transparent light-receiving device, since water does not exist inside the device, heat denaturation, radical damage, decay and the like of a protein the resulting from existence of water are able to be prevented, stability is high, and durability is superior. Further, in the non-wetted all solid protein transparent light-receiving device, since water does not exist inside and outside the device, there is no possibility of electric shock, and intensity is easily secured.

Moreover, in the non-wetted all solid protein transparent light-receiving device, the solid protein layer 43 is directly immobilized onto the transparent electrodes 41 and 42 without a linker molecule or the like in between. Thus, compared to a case that the non-wetted all solid protein transparent light-receiving device is immobilized onto the transparent electrodes 41 and 42 with the linker molecule or the like in between, a larger photocurrent is able to be obtained. Further, in addition to the fact that the solid protein layer 43 is directly immobilized onto the transparent electrodes 41 and 42, the solid protein layer 43 is able to be formed significantly thinly. Thus, the distance between the transparent electrode 41 and the transparent electrode 22 is able to be significantly shortened. Therefore, the non-wetted all solid protein transparent light-receiving device is able to be formed thinly. In addition, by transparentizing the transparent electrodes 41 and 42, a plurality of the non-wetted all solid protein transparent light-receiving devices are able to be laminated. Furthermore, in the non-wetted all solid protein transparent light-receiving device, the size of the electron transfer protein 43a composing the solid protein layer 43 is significantly small, about 2 nm. Thus, for example, it is possible to significantly precisely detect light incidence position in the solid protein layer 43. Thus, a high-definition optical sensor or a high-definition image pickup device is able to be achieved.

Further, it is suspected that photo conductive effect of the electron transfer protein 43a results from "one photon multielectron generation." However, in the liquid protein transparent light-receiving device, since resistance of the solution (solution resistance) existing between the electrodes is high, the foregoing "one photon multielectron generation" is possibly prevented. Meanwhile, in the non-wetted all solid protein transparent light-receiving device, since the solution resistance does not exist, "one photon multielectron generation" is enabled, significant improvement of photoelectric conversion efficiency is able to be improved, and a higher photocurrent is able to be obtained.

The non-wetted all solid protein transparent light-receiving device is able to achieve an optical switching device, an optical sensor, an image pickup device and the like. As described above, since frequency response of the non-wetted all solid protein transparent light-receiving device is fast, the non-wetted all solid protein transparent light-receiving device is able to achieve an optical switching device capable of high-speed switching, a high-speed response optical sensor, an image pickup device capable of capturing an object moving at a high speed and the like. Further, in the case where the non-wetted all solid protein transparent light-receiving device is used for the optical switching device, the optical sensor, the image pickup device and the like, a superior electronic device is able to be achieved.

For example, as will be illustrated later, a camera capable of concurrently focusing on a plurality of objects located in a position different from each other by using one lens is able to be achieved. Further, by using the multilayer transparent light-receiving device, multifocusing and high-speed focusing are enabled with the use of a single lens. Further, in the case where the multilayer transparent light-receiving device is used as a light-receiving device of an optical disc system using a multilayer optical disc or an optical recording reproduction system using a holographic recording medium, parallel readout of the multilayer optical disc and readout of the holographic recording medium are able to be easily performed at a high speed.

<5. Fifth embodiment>

[Multilayer transparent light-receiving device]

A multilayer transparent light-receiving device according to a fifth embodiment has a structure that N layers of the protein transparent light-receiving element 1 are laminated as the multilayer transparent light-receiving device according to the first embodiment. The multilayer transparent light-receiving device according to the fifth embodiment is different from the first embodiment in that multiple pixels composed of the protein transparent light-receiving element 1 are in-plane integrated.

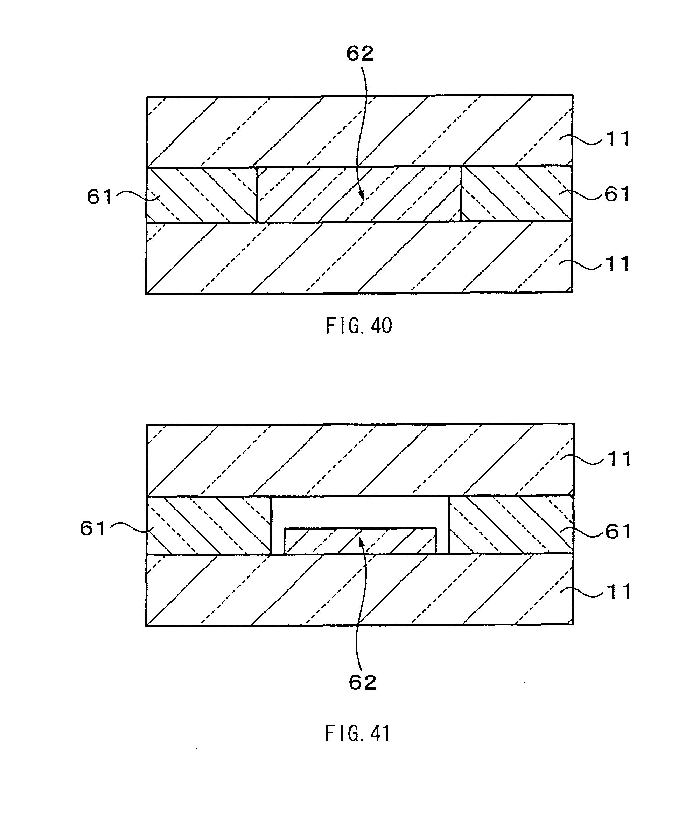

That is, as illustrated in FIG. 40, in the multilayer transparent light-receiving device, for example, a transparent spacer 61 is provided between the Nth transparent substrate 11 and the (N-1)th transparent substrate 11. The distance between these transparent substrates 11 is determined by the thickness of the spacer 61. A pixel 62 composed of the protein transparent light-receiving element 1 is provided in a space between the spacer 61 and the spacer 61, the multiple pixels 62 are in-plane arranged in a state of two dimensional matrix. A face on which the pixels 62 are arranged configures a light receiving face. In total, N stages of the light receiving face exist.

For extracting and processing a signal from the respective pixels 62 in the integrated multilayer transparent light-receiving device and the like, existing known techniques are able to be used. For example, wiring is formed in the line direction and in the column direction to be connected with electrodes above and below the respective pixels 62 arranged in a state of two dimensional matrix in m lines and n columns. In addition, for example, for reading a signal from the m pieces of the pixel 62 in a selected column, a given bias voltage is applied only to wiring connected to one electrode of the pixels 62 in the column, and a photocurrent flown in wiring connected to the other electrode of the pixels 62 in m line is detected.

According to the fifth embodiment, advantages similar to those of the first embodiment are able to be obtained. Further, the integrated multilayer transparent light-receiving device is available for applications similar to those of the multilayer transparent light-receiving device according to the first embodiment.

<6. Sixth embodiment>

[Multilayer transparent light-receiving device]

As illustrated in FIG. 41, in a multilayer transparent light-receiving device according to a sixth embodiment, for example, the transparent spacer 61 with the height variable is provided between the Nth transparent substrate 11 and the (N-1)th transparent substrate 11. The distance between these transparent substrates 11 is determined by the thickness of the spacer 61. In addition, the pixel 62 composed of the protein transparent light-receiving element 1 is provided in a space between the spacer 61 and the spacer 61, and the pixels 62 are in-plane arranged in a state of two dimensional matrix. A face on which the pixels 62 are arranged configures a light receiving face. In total, N stages of the light receiving face exist. In this case, the thickness of the pixel 62 composed of the protein transparent light-receiving element 1 is smaller than the thickness of the spacer 61, the width of the pixel 62 composed of the protein transparent light-receiving element 1 is smaller than the space between the spacer 61 and the spacer 61, a gap exists between the transparent substrate 11 and the pixel 62 and between the spacer 61 and the pixel 62. As described above, since the gap exists between the transparent substrate 11 and the pixel 62 and between the spacer 61 and the pixel 62, the multilayer transparent light-receiving device is able to be configured in a flexible manner.

For extracting and processing a signal from the respective pixels 62 in the integrated multilayer transparent light-receiving device and the like, existing known techniques are able to be used.

According to the sixth embodiment, advantages similar to those of the first embodiment are able to be obtained.

Further, the integrated multilayer transparent light-receiving device is available for applications similar to those of the multilayer transparent light-receiving device according to the first embodiment.

<7. Seventh embodiment>

[Stereoscopic imaging system]

In a stereoscopic imaging system according to a seventh embodiment, a camera including the integrated multilayer transparent light-receiving device according to the fifth embodiment or the sixth embodiment is used as an optical sensor. The camera is a digital camera, a video camera or the like.

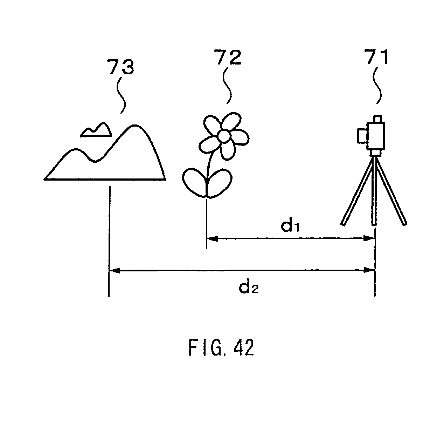

The camera is configured so that the optical axis direction of an image pickup optical system of the camera corresponds with the lamination direction of the pixel 62 composed of the protein transparent light-receiving element 1 of the integrated multilayer transparent light-receiving device. Thereby, in this camera, the respective N stages of light receiving face of the integrated multilayer transparent light-receiving device are able to be used for focusing in capturing an object. Therefore, all objects with each different distance from the camera are able to be focused on and captured. For example, in the case where a flower 72 is located with distance $d_1$ from a camera 71 and a mountain 73 is located with distance $d_2$ ($d_2 > d_1$) from the camera 71 as illustrated in FIG. 42, when the flower 72 and the mountain 73 are captured by the camera 71, both the flower 72 and the mountain 73 are able to be focused on by the integrated multilayer transparent light-receiving device, and are able to be captured in this state. Further, by processing a signal from the integrated multilayer transparent light-receiving device, a three dimensional image is able to be obtained. In the image, both the flower 72 and the mountain 73 are clearly captured. In addition, the flower 72 looks forward, the mountain 73 looks rearward, and perspective is able to be sufficiently obtained.

A description will be given of a case that an image captured by the camera 71 is displayed on a display. In the first example, a realistic three dimensional image captured by the camera 71 is displayed on the display. For example, a realistic three dimensional image in which the flower 71 looks forward and the mountain 72 looks rearward is able to be displayed.

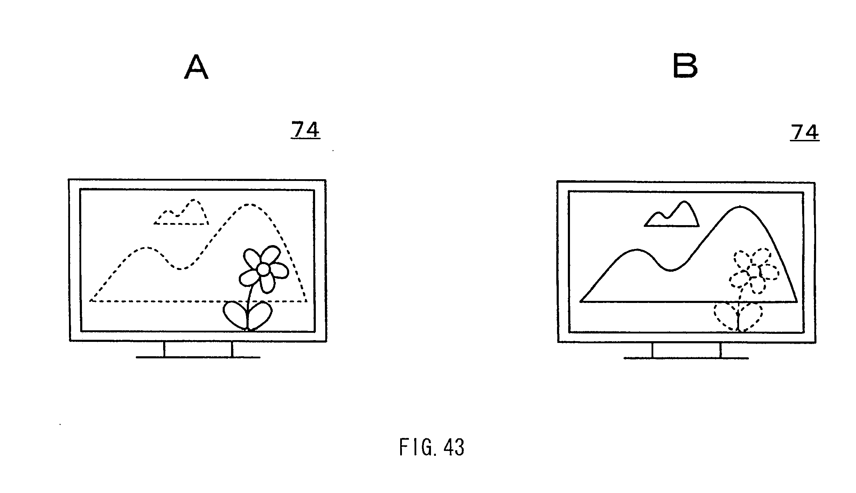

In the second example, a section particularly desired to be viewed in a three dimensional image captured by the camera 71 is displayed emphatically on the display. For example, in the example of FIG. 42, in the case where only the flower 72 is desired to be viewed in the three dimensional image including the flower 72 and the mountain 73 captured by the camera 71, as illustrated in FIG. 43A, it is possible that only the flower 72 is clearly displayed and the mountain 73 is displayed in a blurred manner on a display 74 by processing an image signal. On the other hand, as illustrated in FIG. 43B, it is possible that only the mountain 73 is clearly displayed and the flower 72 is displayed in a blurred manner by processing an image signal. Thereby, an image as desired by a user is able to be displayed on the display 74.

A description will be given of the fact that the respective N stages of light receiving face (faces of the electron transfer protein layer 13) of the integrated multilayer transparent light-receiving device are able to be used for focusing in capturing an object in detail again.

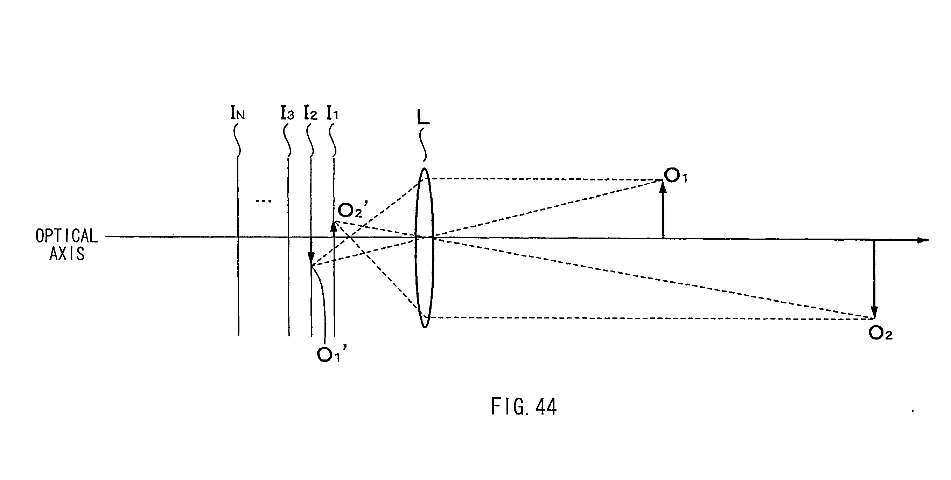

FIG. 44 illustrates a capturing optical system of the integrated multilayer transparent light-receiving device. Though the capturing optical system generally includes two or more lenses, only one lens L exists in this case for convenience of explanation. Fields $I_1$ to $I_N$ correspond to the N stages of light receiving face of the integrated multilayer transparent light-receiving device. Now, the case where objects $O_1$ and $O_2$ with different distance from the lens L exist is considered. An image of the object $O_1$ by the lens L is formed on the field $I_2$ (image point $O_1'$), and an image of the object $O_2$ is formed on the field $I_1$ (image point $O_2'$). In this case, both the objects $O_1$ and $O_2$ are able to be focused on, and clear images thereof are able to be obtained.

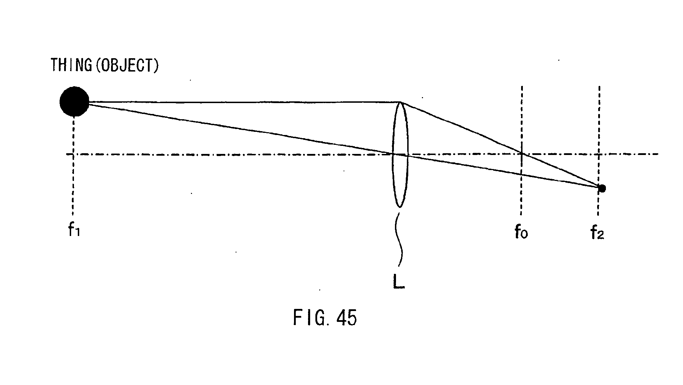

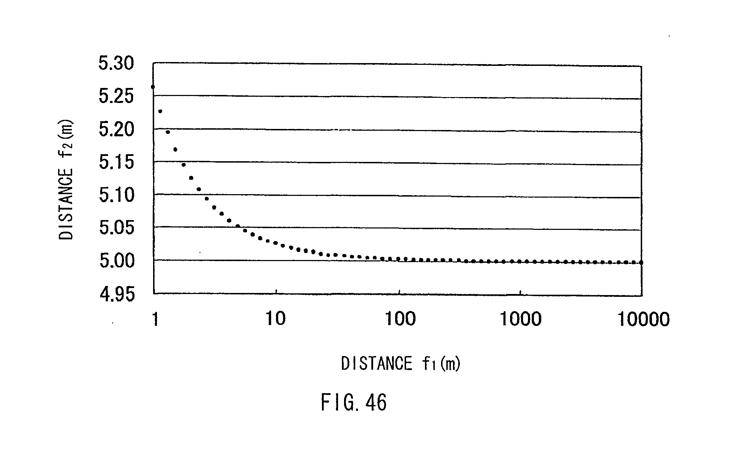

A description will be given of change of position of an imaging face in the integrated multilayer transparent light-receiving device according to distance of an object from the lens L, in other words, change of focus location. As illustrated in FIG. 45, an image of an object with distance $f_1$ from the lens L having a focal length of $f_0$ is formed on position with distance $f_2$ from the lens L. At this time, based on lens formula, $f_1=f_2 f_0/(f_2-f_0)$ is established. As an example, in the case of $f_0$=5 cm, relation between $f_1$ and $f_2$ is as illustrated in Table 5, and is plotted as the graph illustrated in FIG. 46.

[Table 5]

| $f_1$ (m) | $f_2$ (cm) |
|---|---|
| 1 | 5.263158 |
| 5.623413 | 5.044856 |
| 10 | 5.025126 |
| 20.53525 | 5.012204 |
| 56.23413 | 5.00445 |
| 100 | 5.002501 |
| 205.3525 | 5.001218 |
| 562.3413 | 5.000445 |
| 1000 | 5.00025 |
| 10000 | 5.000025 |

As evidenced by Table 5 and FIG. 46, in the case where the distance $f_1$ of the object with from the lens L is changed from 1 m to 10000 m, the distance $f_2$ from the lens L to the object image is changed only by about 0.26 cm. In this case, the space between the first stage of light receiving face and the Nth stage of light receiving face in the integrated multilayer transparent light-receiving device may be 0.3 cm or less.

In the case where the imaging face of the object image by the lens L does not correspond with the light receiving face of the integrated multilayer transparent light-receiving device, in other words, in the case where it is not focused on the light receiving face, an object image is able to be restructured by algorithm of software based on a signal obtained on the respective light receiving faces.

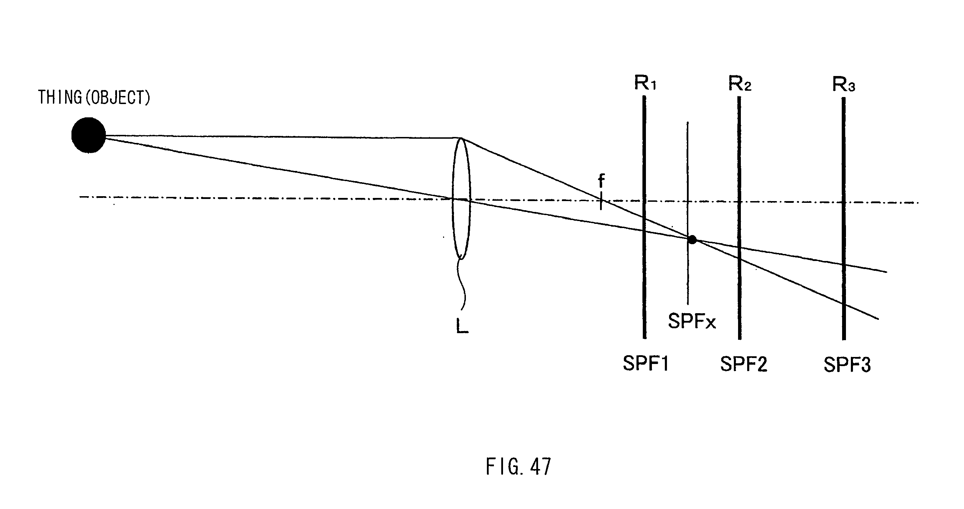

Now, as illustrated in FIG. 47, an object mage that is formed by the lens L is assumed to exist between a light receiving face $R_1$ and a light receiving face $R_2$ out of light receiving faces $R_1$ to $R_3$ of the integrated multilayer transparent light-receiving device. In this case, as function F (SPF1, SPF2, and SPF3) of point spread function SPF1, SPF2, and SPF3 in the respective light receiving faces $R_1$ to $R_3$, point spread function $SPF_x$ of the imaging face of the object is able to be obtained. Such calculation is able to be easily performed by a computer. Further, the object image is able to be obtained by using the point spread function $SPF_x$, and such an image is able to be displayed on a display.

For example, by using the foregoing technique in capturing by a television camera in a broadcast station, in the case where an image is displayed on a three dimensional television by using a video signal transmitted from the broadcast station, a portion particularly desired to be viewed by a user in the displayed images is able to be freely zoomed in or zoomed out based on an output signal from the light receiving face of the integrated multilayer transparent light-receiving device.

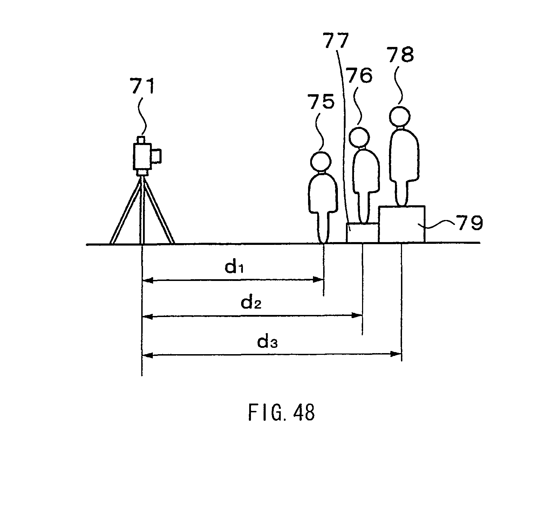

By using the camera 71, a clear image of a plurality of things (objects) with each distance different from each other from the camera 71 is able to be concurrently obtained. For example, as illustrated in FIG. 48, a case where a person 75 in the first row stands on the ground, a person 76 in the second row stands on a low stool 77, a person 78 in the third row stands on a stool 79 higher than the stool 77, and the persons 75, 76, and 78 are captured by the camera 71 is considered. In this case, the persons 75, 76, and 78 are able to be respectively focused on by the multilayer transparent light-receiving device of the camera 71. Thus, a clear image of the persons 75, 76, and 78 is able to be concurrently obtained.

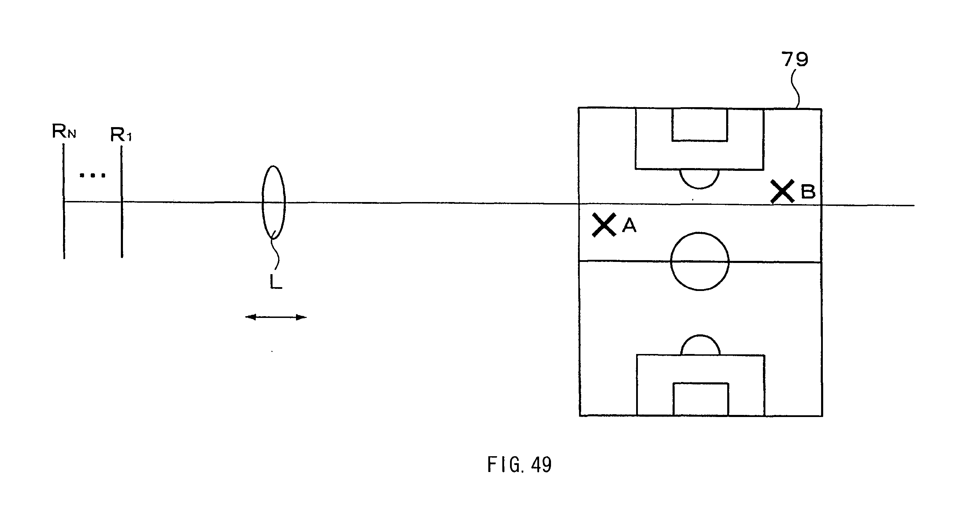

By using the camera 71, an object desired to be captured is able to be focused on at high velocities. For example, a case that a soccer game is held in a soccer coat 79 and the game is captured by the camera 71 as illustrated in FIG. 49 is considered. Now, from a state that point A is focused on in the soccer coat 79, point B will be focused on. In this case, in the case where a general camera is used, a lens of the camera should be moved largely. Meanwhile, in the case where the camera 71 is used, the point B is able to be focused on without moving the lens L much, and focusing is able to be made at high velocities for the following reason.

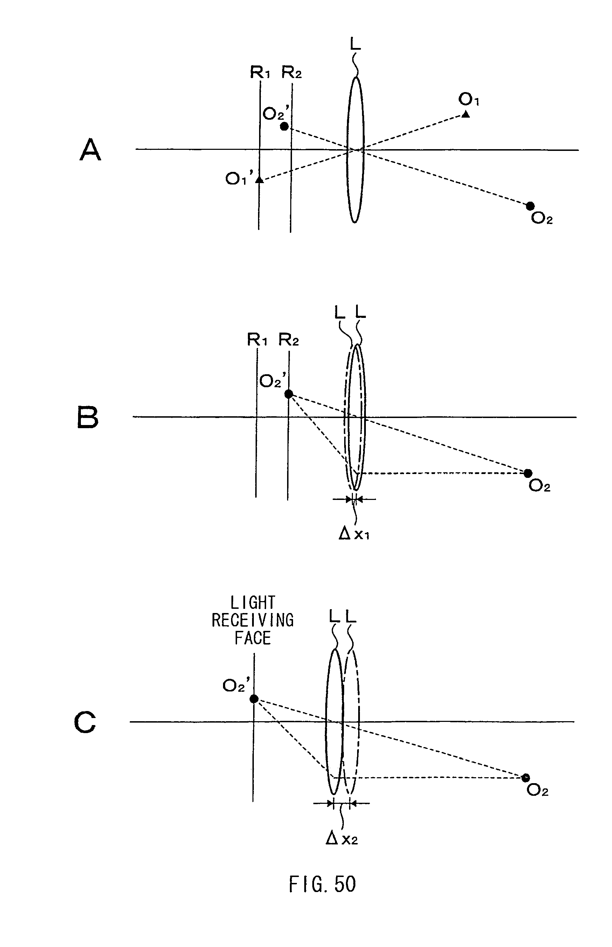

That is, as illustrated in FIG. 50A, first, thing $O_1$ in the point A of the soccer coat 79 is focused on, image $O_1$' is formed on the light receiving face R1 of the integrated multilayer transparent light-receiving device of the camera 71, thing $O_2$ in the point B is not focused on, and image $O_2$' is formed on location slightly shifted from the light receiving face $R_2$ of the integrated multilayer transparent light-receiving device of the camera 71. In the case where the point B is focused on from such a state, in an existing camera, as illustrated in FIG. 50C, it is necessary to move the lens L by distance $\Delta_{x2}$ corresponding to position difference between the image $O_1$' and the image $O_2$' and thereby the image $O_2$' of the thing $O_2$ is formed on the light receiving face. Meanwhile, in the case where the camera 71 is used, as illustrated in FIG. 50B, it is enough to move the lens L by only distance $\Delta_{x1}$ between the image $O_2$' and the light receiving face $R_2$ so that the image $O_2$' is formed on the light receiving face $R_2$ adjacent to the light receiving face $R_1$. Therefore, movement distance of the lens L may be small, and thus focusing on the point B is able to be performed at high velocities. Further, the camera 71 is able to be configured more thinly.

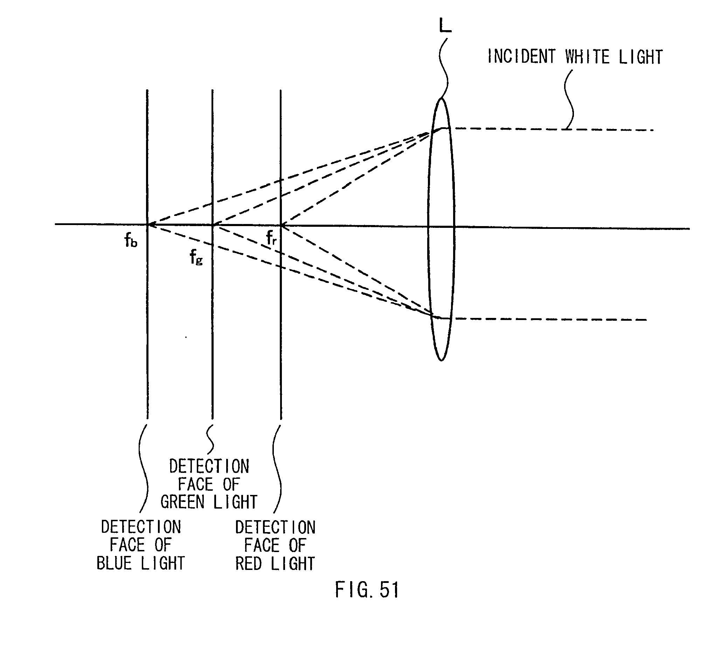

By using the camera 71, chromatic aberration is able to be corrected without using an expensive achromatic lens. That is, as illustrated in FIG. 51, in the case where white light enters the lens L, even if, for example, blue light, green light, and red light form an image on each different face (each distance from the lens L is $f_b$, fg, and $f_r$) due to chromatic aberration of the lens L, the blue light, the green light, and the red light are able to be received on one of the light receiving faces $R_1$ to $R_N$ of the integrated multilayer transparent light-receiving device of the camera 71.

<8. Eighth embodiment>

[Stereoscopic imaging system]

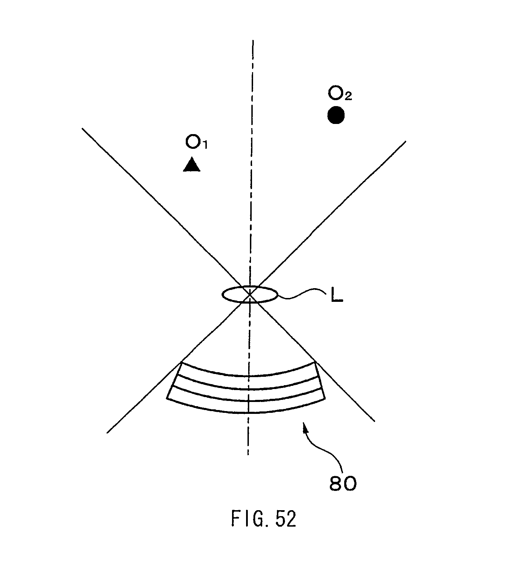

In a stereoscopic imaging system according to an eighth embodiment, a camera including the integrated multilayer transparent light-receiving device according to the sixth embodiment is used as an optical sensor. As illustrated in FIG. 52, in the camera 71, an integrated multilayer transparent light-receiving device 80 in the curved shape is used as an optical sensor. In addition, the lens L is arranged in the vicinity of the center of curvature of the integrated multilayer transparent light-receiving device 80. Thereby, a plurality of things located in a wide angle range (for example, the things $O_1$ and $O_2$) are able to be captured concurrently.

<9. Ninth embodiment>

[Stereoscopic imaging system]

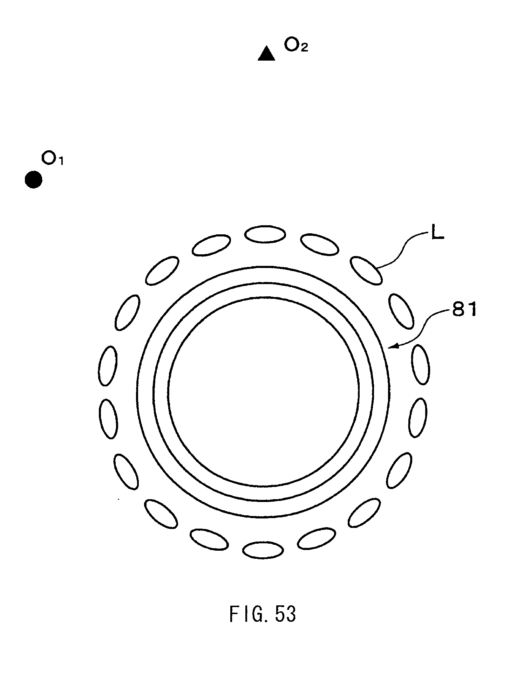

In a stereoscopic imaging system according to a ninth embodiment, a camera including the integrated multilayer transparent light-receiving device according to the sixth embodiment is used as a light-receiving device. As illustrated in FIG. 53, in the camera 71, an integrated multilayer transparent light-receiving device 81 in the columnar shape is used as a light-receiving device. In addition, the lens L is arranged in the outer circumference of the integrated multilayer transparent light-receiving device 81. Thereby, the things $O_1$ and $O_2$ located in 360 deg range are able to be captured concurrently, and all-round stereoscopic imaging system is able to be obtained.

<10. Tenth embodiment>

[Optical disc system]

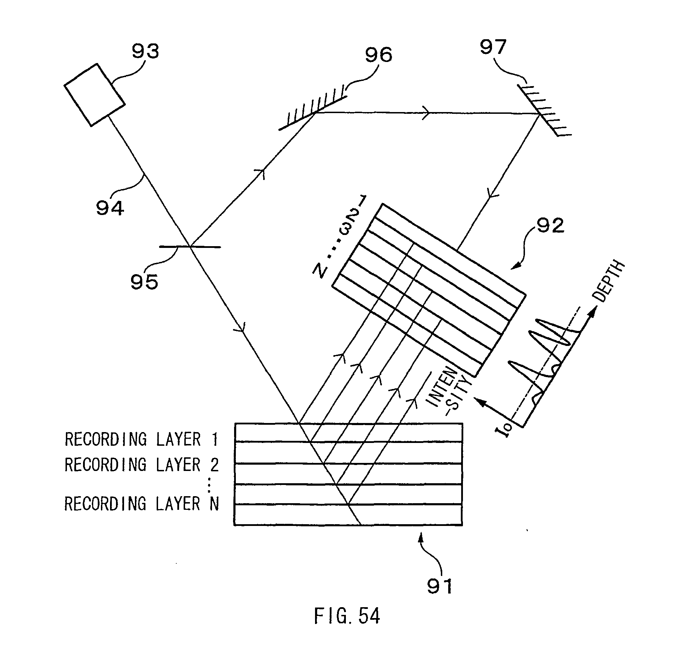

FIG. 54 illustrates an optical disc system according to a tenth embodiment.
As illustrated in FIG. 54, in the optical disc system, a multilayer optical disc 91 having N layers of recording layer is used, a multilayer transparent light-receiving device 92 having N layers of the protein transparent light-receiving element 1 is used, and thereby digital data recorded on the N layers of recording layer of the multilayer optical disc 91 is read out in batch. Specifically, as illustrated in FIG. 54, light 94 from a light source 93 with low coherence is divided into two by a beam splitter 95, and light transmitted through the beam splitter 95 enters the multilayer optical disc 91. The light entering the multilayer optical disc 91 is respectively reflected by each recording layer, and enters the multilayer transparent light-receiving device 92. Meanwhile, light reflected by the beam splitter 95 is sequentially reflected by mirrors 96 and 97, and subsequently enters the multilayer transparent light-receiving device 92. In the case where the light that has been divided into two by the beam splitter 95 enters the multilayer transparent light-receiving device 92, such light generates interference. In the result, light intensity distribution in the light receiving face of N layers of the multilayer transparent light-receiving device 92 is obtained as illustrated in the section right next to the multilayer transparent light-receiving device 92 of FIG. 54. The intensity distribution reflects the data recorded in the respective recording layers of the multilayer optical disc 91. In this case, for example, by setting a state that intensity peak is higher than threshold intensity $I_0$ to "1" and setting a state that intensity peak is lower than threshold intensity $I_0$ to "0", the digital data recorded on the multilayer optical disc 91 is able to be read out.

<11. Eleventh embodiment>

[Optical recording reproduction system]

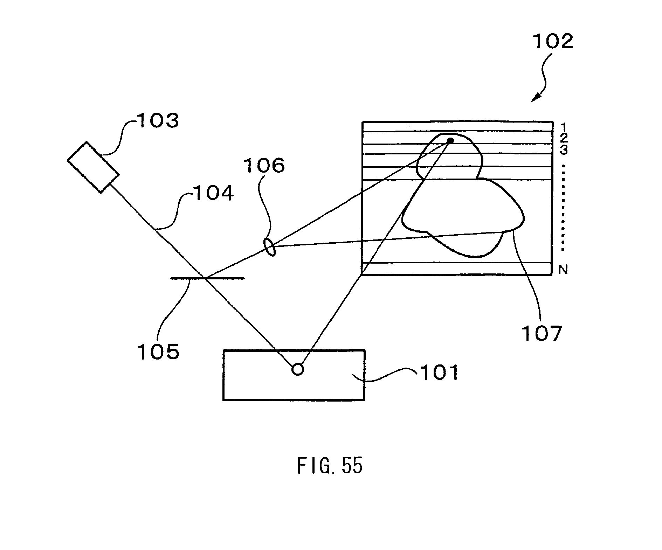

FIG. 55 illustrates an optical recording reproduction system according to an eleventh embodiment.
As illustrated in FIG. 55, in the optical recording reproduction system, a holographic recording medium 101 is used, a multilayer transparent light-receiving device 102 having N layers of the protein transparent light-receiving element 1 is used, and thereby data recorded on the holographic recording medium 101 is read out. Specifically, as illustrated in FIG. 55, light 104 from a light source 103 with high coherence is divided into two by a beam splitter 105, and light transmitted through the beam splitter 105 enters the holographic recording medium 101. The light entering the holographic recording medium 101 is directed to the multilayer transparent light-receiving device 92. Meanwhile, light reflected by the beam splitter 105 enters the multilayer transparent light-receiving device 102 through a lens 106, and is overlapped on light coming from the holographic recording medium 101. In the result, an image recorded on the holographic recording medium 101 is shown on the multilayer transparent light-receiving device 92 as light intensity distribution. Accordingly, the image recorded in the holographic recording medium 101 is able to be reproduced.
The present invention has been specifically described with reference to the embodiments and the example of the present invention. However, the present invention is not limited to the foregoing embodiments and the foregoing example, and various modifications may be made based on the technical idea of the present invention.
For example, the numerical values, the structures, the configurations, the shapes, the materials and the like described in the foregoing embodiments and the foregoing example are only examples. Numerical values, structures, configurations, shapes, materials and the like different from those described in the foregoing embodiments and the foregoing example are able to be used according to needs.

**Claims**

1. A multilayer transparent light-receiving device having a plurality of protein transparent light-receiving elements laminated on each other and using an electron transfer protein.

2. The multilayer transparent light-receiving device according to claim 1, wherein the electron transfer protein is tin-substituted cytochrome c obtained by substituting iron as a central metal of heme of mammal-derived cytochrome c with tin, or a protein that is composed of an amino-acid sequence obtained by losing, substituting, or adding one or several amino acids in an amino-acid sequence of the mammal-derived cytochrome c and that contains tin.

3. The multilayer transparent light-receiving device according to claim 2, wherein the mammal-derived cytochrome c is horse heart cytochrome c or bovine heart cytochrome c.

4. The multilayer transparent light-receiving device according to claim 1, wherein the electron transfer protein is immobilized onto a transparent electrode.

5. The multilayer transparent light-receiving device according to claim 4, wherein the protein transparent light-receiving element has a protein immobilized electrode in which the electron transfer protein is immobilized onto the transparent electrode, and a counter electrode.

6. The multilayer transparent light-receiving device according to claim 1, wherein the protein transparent light-receiving element has a structure in which a solid protein layer composed of the electron transfer protein is sandwiched between a first electrode and a second electrode.

7. An electronic device comprising a multilayer transparent light-receiving device that has a plurality of protein transparent light-receiving elements laminated on each other, using an electron transfer protein.

8. The electronic device according to claim 7, wherein the electron transfer protein is tin-substituted cytochrome c obtained by substituting iron as a central metal of heme of mammal-derived cytochrome c with tin, or a protein that is composed of an amino-acid sequence obtained by losing, substituting, or adding one or more amino acids in an amino-acid sequence of the mammal-derived cytochrome c and that contains tin.

9. The electronic device according to claim 8, wherein the mammal-derived cytochrome c is horse heart cytochrome c or bovine heart cytochrome c.

10. The electronic device according to claim 7, wherein the electron transfer protein is immobilized onto a transparent electrode.

11. The electronic device according to claim 10, wherein the protein transparent light-receiving element has a protein immobilized electrode in which the electron transfer protein is immobilized onto the transparent electrode, and a counter electrode.

12. The electronic device according to claim 7, wherein the protein transparent light-receiving element has a structure in which a solid protein layer composed of the electron transfer protein is sandwiched between a first electrode and a second electrode.

13. The electronic device according to claim 7, wherein the electronic device is a three dimensional display, a three dimensional image sensor, a camera, or an optical recording reproduction system.

FIRST LAYER ⟨1

SECOND LAYER ⟨1

⋮ ⟨1

Nth LAYER ⟨1

FIG. 1

1

⟨15

⟨14

⟨13

⟨12

⟨11

FIG. 2

FIG. 3

19a    19b

16

14

12  13    15

FIG. 4

15
14
13
12

20

FIG. 5

FIG. 6

FIG. 7

FIG. 8

FIG. 9

FIG. 10

TIN-SUBSTITUTED HORSE
HEART CYTOCHROME c

WAVELENGTH (nm)

ABSORBANCE

EP 2 498 302 A1

FIG. 11

FIG. 12

ZINC-SUBSTITUTED BOVINE HEART CYTOCHROME c

0 MINUTES
AFTER 30 MINUTES
AFTER 60 MINUTES
AFTER 90 MINUTES
AFTER 120 MINUTES

ABSORBANCE

WAVELENGTH (nm)

FIG. 13

FIG. 14

FIG. 15

FIG. 16

FIG. 17

FIG. 18

FIG. 19

FIG. 20

FIG. 21

EP 2 498 302 A1

FIG. 22

FIG. 23

FIG. 24

FIG. 25

A

52

52a

51

52b

B

53

52a

52

51

52b

FIG. 26

A

52a ——— 43

51

52 ———

52b

B

54

52a ——— 43

51

52 ———

55

52b

FIG. 27

FIG. 28

FIG. 29

FIG. 30

FIG. 31

FIG. 32

FIG. 33

FIG. 34

FIG. 35

FIG. 36

FIG. 37

FIG. 38

FIG. 39

FIG. 40

FIG. 41

73 72 71

d₁

d₂

FIG. 42

FIG. 43

FIG. 44

THING (OBJECT)

$f_1$          $f_0$     $f_2$

L

FIG. 45

FIG. 46

THING(OBJECT)

R₁　　　R₂　　　R₃

f

L

SPFx

SPF1　　SPF2　　SPF3

FIG. 47

EP 2 498 302 A1

FIG. 48

FIG. 49

FIG. 50

EP 2 498 302 A1

L

INCIDENT WHITE LIGHT

$f_b$

$f_g$

$f_r$

DETECTION
FACE OF
GREEN LIGHT

DETECTION
FACE OF
BLUE LIGHT

DETECTION
FACE OF
RED LIGHT

FIG. 51

O₂

O₁

L

80

FIG. 52

FIG. 53

FIG. 54

FIG. 55

## INTERNATIONAL SEARCH REPORT

| | International application No. |
|---|---|
| | PCT/JP2010/069197 |

**A. CLASSIFICATION OF SUBJECT MATTER**
*H01L31/10*(2006.01)i, *C07K17/02*(2006.01)i

According to International Patent Classification (IPC) or to both national classification and IPC

**B. FIELDS SEARCHED**

Minimum documentation searched (classification system followed by classification symbols)
H01L31/10, C07K17/02

Documentation searched other than minimum documentation to the extent that such documents are included in the fields searched
Jitsuyo Shinan Koho          1922-1996    Jitsuyo Shinan Toroku Koho    1996-2011
Kokai Jitsuyo Shinan Koho    1971-2011    Toroku Jitsuyo Shinan Koho    1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2007-220445 A  (Sony Corp.), 30 August 2007 (30.08.2007), entire text; all drawings & US 2008/0000525 A1     & EP 1821324 A2 & KR 10-2007-0082519 A   & CN 101055919 A | 1-13 |
| Y | JP 2-281770 A  (Director General, Agency of Industrial Science and Technology), 19 November 1990 (19.11.1990), page 3, upper left column to page 4, upper left column; fig. 1 (Family: none) | 1-13 |

☒  Further documents are listed in the continuation of Box C.          ☐  See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T"  later document published after the international filing date or priority date and not in conflict with the application but cited to understand the principle or theory underlying the invention

"X"  document of particular relevance; the claimed invention cannot be considered novel or cannot be considered to involve an inventive step when the document is taken alone

"Y"  document of particular relevance; the claimed invention cannot be considered to involve an inventive step when the document is combined with one or more other such documents, such combination being obvious to a person skilled in the art

"&"  document member of the same patent family

| Date of the actual completion of the international search | Date of mailing of the international search report |
|---|---|
| 03 February, 2011 (03.02.11) | 15 February, 2011 (15.02.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/069197

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 63-237585 A  (Mitsubishi Electric Corp.), 04 October 1988 (04.10.1988), page 2, upper left column to page 5, upper right column; fig. 1 to 5 (Family: none) | 1-13 |
| Y | Eur. J. Biochem., Vol.64, No.2, pp.381-387 | 1-13 |
| Y | Biochemistry 1987, Vol.26, No.11, pp.3142-3148 | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002334986 A

- JP 2007220445 A

**Non-patent literature cited in the description**

- **MCLENDON G. ; SMITH M.** J. *Biol. Chem.,* 1978, vol. 253, 4004
- **MOZA B.** *Biochim. Biophys. Acta,* 2003, vol. 1646, 49
- **VANDERKOOI, J. M.** *Eur. J. Biochem.,* 1976, vol. 64, 381-387

- **TOKITA, Y.** *J. Am. Chem. Soc.,* 2008, vol. 130, 5302
- Optical spectra and electronic structure of porphyrins and related rings. **GOUTERMAN M.** The Porphyrins. Academic Press, 1978, vol. 3, 1-156